# EUROPÄISCHE PATENTSCHRIFT

(11) **EP 1 209 164 B1**
(45) Veröffentlichungstag und Bekanntmachung des Hinweises auf die Patenterteilung: **10.12.2003**
(21) Anmeldenummer: 01124864.8
(22) Anmeldetag: 18.10.2001
(51) Int. Cl.: C07F 9/6584, C07F 15/00, C07C 45/50, B01J 31/18

(54) **Neue Phosphininverbindung und deren Metallkomplexe**
Novel phosphinine compounds and their metal complexes
Nouveaux composés de type phosphinine et leurs complexes de métaux

(30) Priorität: 24.11.2000 DE 10058383
(43) Veröffentlichungstag der Anmeldung: 29.05.2002
(73) Patentinhaber: Oxeno Olefinchemie GmbH, 45772 Marl (DE)
(72) Erfinder: Röttger, Dirk, Dr., 45657 Recklinghausen (DE); Hess, Dieter, Dr., 45770 Marl (DE); Börner, Armin, Prof. Dr., 18059 Rostock (DE); Selent, Detlef, Dr., 10318 Berlin (DE); Kadyrov, Renat, Dr., 65931 Frankfurt (DE); Wiese, Klaus-Dieter, Dr., 45721 Haltern (DE); Borgmann, Cornelia, Dr., 45657 Recklinghausen (DE)

(56) Entgegenhaltungen:
- EP-A- 0 214 622
- EP-A- 0 247 595
- EP-A- 0 472 071
- DE-A- 19 602 301
- LEVINA E. YA. WT AL.: "1,2-dihydro-1,2- azaphosphorines from the reactions of phosphorus trichloride and of ethylphosphonous dichloride with N-(2-ethylhexylidene)butylamine" RUSSIAN JOURNAL OF GENERAL CHEMISTRY., Bd. 60, Nr. 4, - 20. September 1990 (1990-09-20) Seiten 663-670, XP002190244 CONSULTANTS BUREAU., US ISSN: 1070-3632

## Beschreibung

Die vorliegende Erfindung betrifft Phosphinine und deren Metallkomplexe, die Herstellung, sowie die Verwendung der Phosphinine als Liganden in katalytischen Reaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung (Oxierung) bekannt. Als Katalysatoren in diesen Reaktionen werden häufig Verbindungen der Übergangsmetalle der VIII. Gruppe des Periodensystems der Elemente verwendet, insbesondere Rhodium- und Kobaltverbindungen. Die Hydroformylierung mit Rhodiumverbindungen bietet im Vergleich zur Katalyse mit Kobalt in der Regel den Vorteil höherer Selektivität und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite. Eine gute Übersicht über den Stand der Hydroformylierung von Olefinen findet sich in B. CORNILS, W. A. HERRMANN, *"Applied Homogeneous Catalysis with Organometallic Compounds"*, Vol. 1&2, VCH, Weinheim, New York, 1996.

Jedes Katalysatorsystem (Kobalt oder Rhodium) hat seine spezifischen Vorzüge. Je nach Einsatzstoff und Zielprodukt kommen daher unterschiedliche Katalysatorsysteme zum Einsatz, wie folgende Beispiele zeigen. Arbeitet man mit Rhodium und Triphenylphosphin, lassen sich α-Olefine bei niedrigeren Drücken hydroformylieren. Als Phosphor-haltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zweizähnigen Phosphinliganden ist ein relativ hoher Aufwand, der zu ihrer Darstellung notwendig ist. Daher ist es oftmals nicht rentabel, solche Systeme in technischen Prozessen einzusetzen.

Rhodium-Monophosphit-Komplexe sind geeignete Katalysatoren für die Hydroformylierung von verzweigten Olefinen mit innenständigen Doppelbindungen, jedoch ist die Selektivität für endständig oxierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten bekannt.

Die DE-A-196 02 301 beschreibt Bisphosphit-Verbindungen und die Verwendung deren Metallkomplexe in katalytischen Reaktionen, insbesondere für die Hydroformylierung von Olefinen.

Rhodium-Bisphosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen nur im geringen Maße umgesetzt. Diese Liganden ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyseempfindlichkeit der Phosphitliganden, unbefriedigend. Durch den Einsatz von substituierten Bisaryldiolen als Edukte für die Phosphitliganden, wie in EP 0 214 622 oder EP 0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In den Patenten US 4 668 651, US 4 748 261 und US 4 885 401 werden Polyphosphitliganden beschrieben, mit denen α-Olefine, aber auch 2-Buten mit hoher Selektivität zu den terminal oxierten Produkten umgesetzt werden können. Zweizähnige Liganden dieses Typs wurden auch zur Hydroformylierung von Butadien eingesetzt (US 5 312 996).

Obgleich die genannten Phosphinine sehr gute Komplexliganden für Rhodium-Hydroformylierungskatalysatoren sind, ist es wünschenswert, deren Wirksamkeit und Stabilität noch weiter zu verbessern.

Es wurde gefunden, dass Phosphinine mit den allgemeinen Struktur I 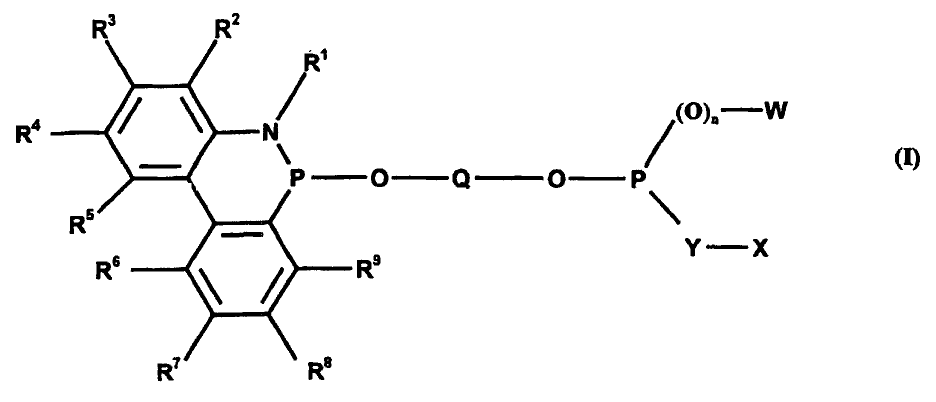 einfach hergestellt werden können und als Liganden bei Metall-katalysierten Reaktionen geeignet sind.

Gegenstand der vorliegenden Erfindung sind daher Phosphinine der allgemeinen Formel I 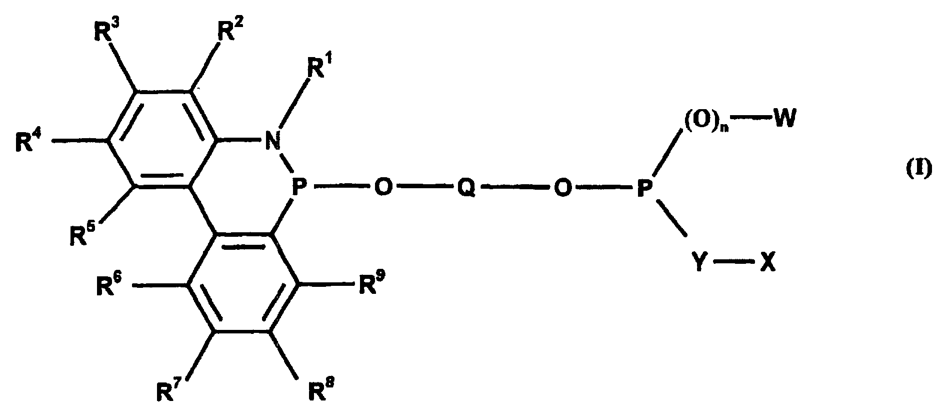 mit
n = 0 oder 1,
Y = O, NH, NR¹,
R¹ = H, aliphatischer oder aromatischer Kohlenwasserstoffrest mit 1 bis 25 Kohlenstoffatomen, CH₃,
R², R³, R⁴, R⁵, R⁶, R⁷, R⁸, R⁹ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R² bis R⁹ eine gleiche oder unterschiedliche Bedeutung besitzen und kovalent miteinander verknüpft sein können,
F, Cl, Br, I, -CF₃, -OR¹⁰, -COR¹⁰, -CO₂R¹⁰, -CO₂M, -SR¹⁰, -SO₂R¹⁰, -SOR¹⁰, -SO₃R¹⁰, -SO₃M, -SO₂NR¹⁰R¹¹, NR¹⁰R¹¹, N=CR¹⁰R¹¹, NH₂,
R¹⁰, R¹¹ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, die gleich oder unterschiedlich oder kovalent miteinander verknüpft sein können.
Spezielle Ausführungsformen der erfindungsgemäßen Phosphinine betreffen Phosphinine der Formeln II, III, IV und V 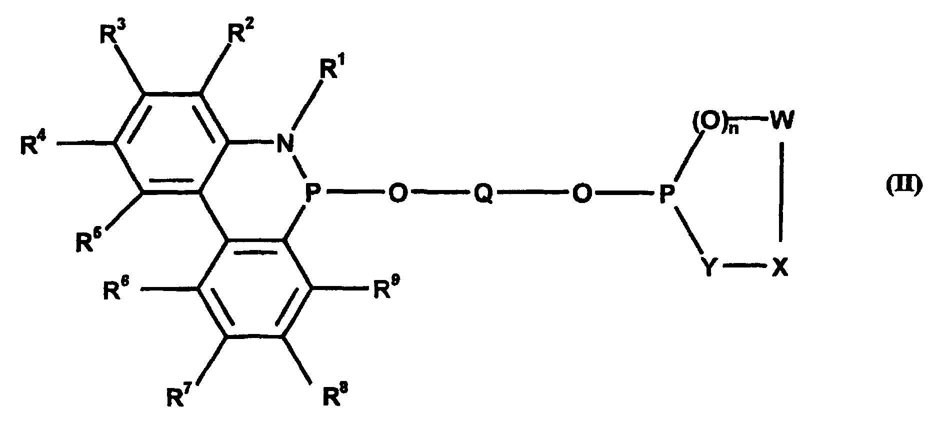 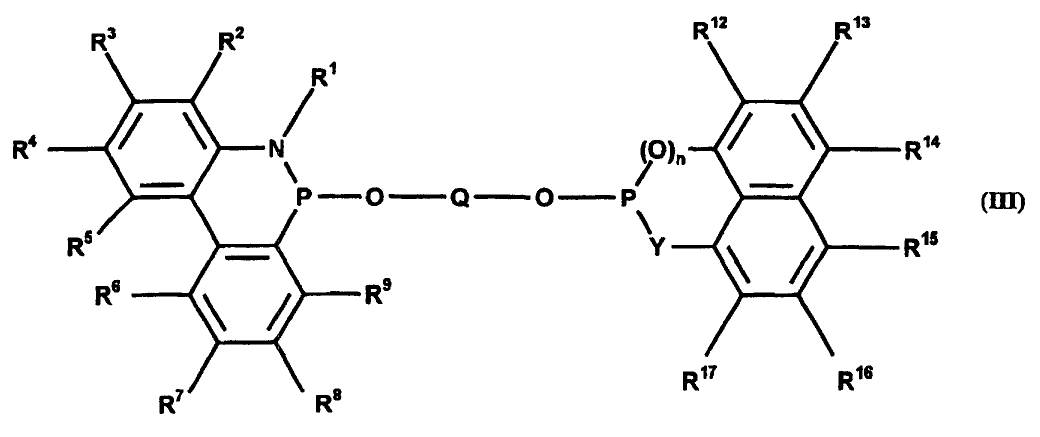 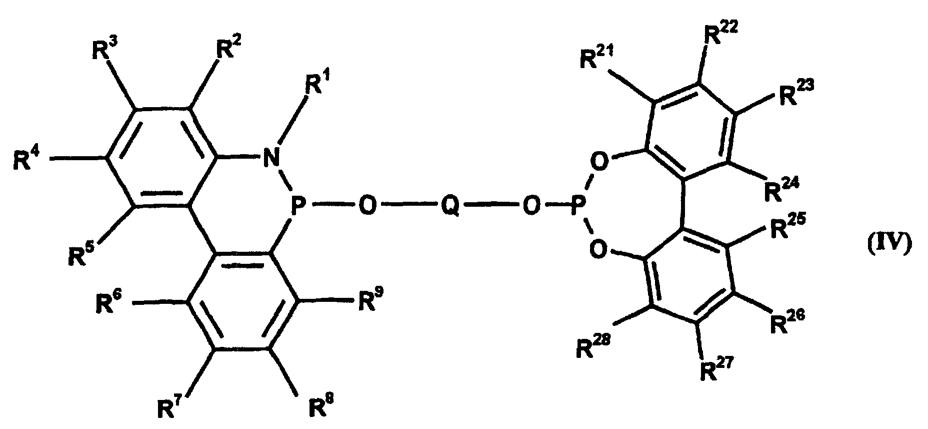 und

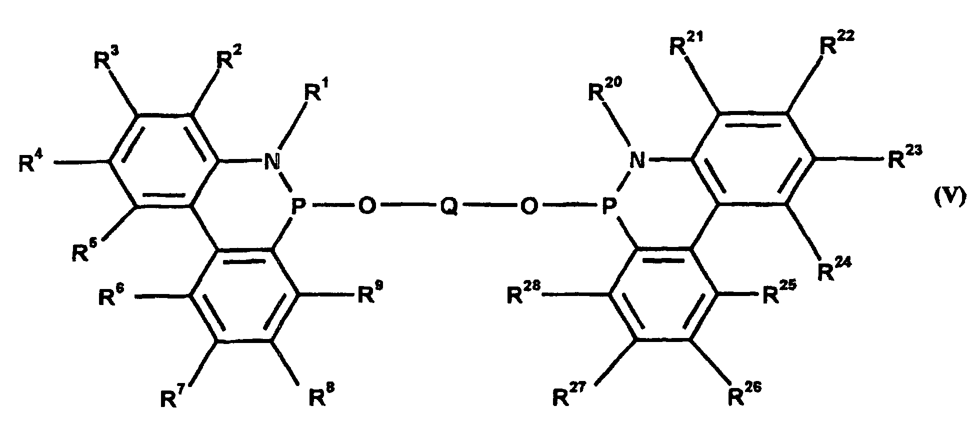

Wobei für Formel II W und X aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen bedeuten, X und W gleich oder unterschiedlich und kovalent mit einander verknüpft sein können. X und W stehen in den Formeln III, IV und V für die bezeichneten substituierten oder unsubstituierten Bisphenyl- oder Naphthylsysteme, Y steht für O, NH, NR¹, insbesondere für Sauerstoff, n für 0 oder 1. Die funktionellen Reste R¹ bis R³⁰ und Q besitzen die folgende oder die bereits genannten Bedeutungen.

In Formel III stehen R¹², R¹³, R¹⁴, R¹⁵, R¹⁶, R¹⁷ für H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R¹² bis R¹⁷ eine gleiche oder unterschiedliche Bedeutung besitzen und kovalent miteinander verknüpft sein können,
F, Cl, Br, I, -CF₃, -OR¹⁸, -COR¹⁸, -CO₂R¹⁸, -CO₂M, -SR¹⁸, -SO₂R¹⁸, -SOR¹⁸, -SO₃R¹⁸, -SO₃M, -SO₂NR¹⁸R¹⁹, NR¹⁸R¹⁹, N=CR¹⁸R¹⁹, NH₂,
M steht für ein Alkalimetall-, Erdalkalimetall-, Ammonium-, oder Phosphoniumion.
R¹⁸ und R¹⁹ können gleich oder unterschiedlich sein und jeweils für H, Methyl, t-Butyl, substituierte oder unsubstituierte, aliphatische oder aromatische Kohlenwasserstoffreste mit 1 bis 25 Kohlenstoffatomen, mit gleicher oder unterschiedlicher Bedeutung stehen.

In Formel IV bedeuten:
R²¹, R²², R²³, R²⁴, R²⁵, R²⁶, R²⁷, R²⁸ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R²¹ bis R²⁸ eine gleiche oder unterschiedliche Bedeutung besitzen und kovalent miteinander verknüpft sein können,
F, Cl, Br, I, CF₃, -OR²⁹, -COR²⁹, -CO₂R²⁹, -CO₂M, -SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, substituierter oder unsubstituierter, aliphatischer oder aromatischer Kohlenwasserstoffrest mit 1 bis 25 Kohlenstoffatomen,
M = Alkalimetall, Erdalkalimetall, Ammonium, Phosphoniumion,
Y bedeutet Sauerstoff,
n besitzt den Wert 1
und
R¹, R², R³, R⁴, R⁵, R⁶, R⁷, R⁸, R⁹ und Q tragen die in Anspruch 1 genannten Bedeutungen, wobei R² bis R⁹ eine gleiche oder unterschiedliche Bedeutung besitzen und kovalent miteinander verknüpft sein können.

In Formel V stehen R²⁰ für H, CH₃, aliphatischer oder aromatischer Kohlenwasserstoffrest mit 1 bis 25 Kohlenstoffatomen,
R²¹, R²², R²³, R²⁴, R²⁵, R²⁶, R²⁷, R²⁸ für H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R²¹ bis R²⁸ eine gleiche oder unterschiedliche Bedeutung besitzen und kovalent miteinander verknüpft sein können,
F, Cl, Br, I, CF₃, -OR²⁹, -COR²⁹, -CO₂R²⁹, -CO₂M, -SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, substituierter oder unsubstituierter, aliphatischer oder aromatischer Kohlenwasserstoffrest mit 1 bis 25 Kohlenstoffatomen,
M = Alkalimetall, Erdalkalimetall, Ammonium, Phosphoniumion,
Y bedeutet NR²⁰,
n besitzt den Wert 0
und
R¹, R², R³, R⁴, R⁵, R⁶, R⁷, R⁸, R⁹ und Q tragen die in Anspruch 1 genannten Bedeutungen, wobei R² bis R⁹ eine gleiche oder unterschiedliche Bedeutung besitzen und kovalent miteinander verknüpft sein können.

Beispiele für Q sind bivalente Kohlenwasserstoffreste, die aliphatisch, alicyclisch, aliphatisch-alicyclisch, heterocyclisch, aliphatisch-heterocylisch, aromatisch oder aliphatisch-aromatisch sein können. Gegebenenfalls vorhandene Ringsysteme können ihrerseits mit den oben genannten Kohlenwasserstoffresten substituiert sein. In offenkettigen Strukturelementen können eine oder mehrere Methylengruppen durch Sauerstoff und/oder Schwefel und/oder NR¹ und/oder NH und/oder eine oder mehrere CH-Gruppen durch Stickstoff ersetzt sein.

Bevorzugt steht Q für bivalente Reste, die aromatische Gruppen enthalten. Q kann beispielsweise ein Phenylenrest, Naphthylenrest, ein zweiwertiger Bisarylenrest oder ein bivalenter Rest eines Diphenylethers sein. Weiterhin kann Q die allgemeine Struktur -Ar-Z-Ar- haben. Darin bedeutet Ar einen einringigen oder mehrringigen bivalenten aromatischen Rest. Z steht entweder für eine direkte Bindung oder für eine gegebenenfalls substituierte Methylengruppe -CR⁴¹R⁴²-, wobei R⁴¹ und R⁴² für Wasserstoff und/oder aliphatische und/oder aromatische Reste mit 1 bis 25 Kohlenstoffatomen stehen und die darüber hinaus Heteroatome enthalten können. Weiterhin können die Reste R⁴¹ und R⁴² zu einem oder mehreren Ringen verknüpft sein, d. h. eine kovalente Bindung aufweisen.
Von den Phosphininen nach den allgemeinen Formeln I, II, III, IV und V sind diejenigen besonders bevorzugt, bei denen der Rest Q für einen Kohlenwasserstoffrest (Bisarylenrest) nach der allgemeinen Formel VI steht 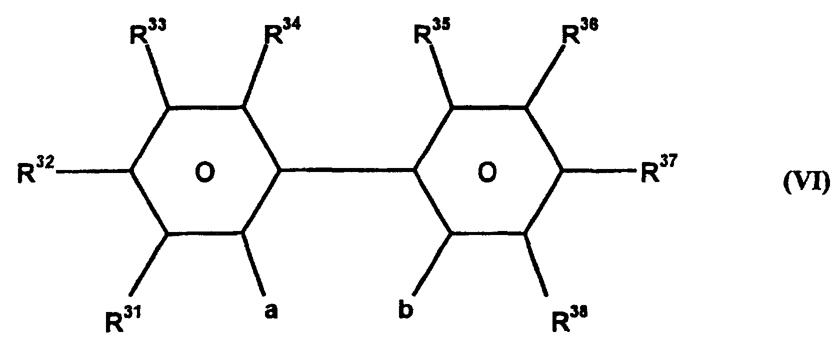 mit
R³¹, R³², R³³, R³⁴, R³⁵, R³⁶, R³⁷, R³⁸ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, CF₃, -OR³⁹, -COR³⁹, -CO₂R³⁹, -CO₂M, -SR³⁹, - SO₂R³⁹, -SOR³⁹, -SO₃R³⁹, -SO₃M, -SO₂NR³⁹R⁴⁰, NR³⁹R⁴⁰, N=CR³⁹R⁴⁰, NH₂,
R³⁹, R⁴⁰ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion,
wobei die Positionen a und b als Anknüpfpunkte dieses Substituenten im Strukturelement O-Q-O in den Verbindungen der Formeln I, II, III, IV und V stehen.

Beispiele für W und X sind Kohlenwasserstoffreste, die aliphatisch, alicyclisch, aliphatisch-alicyclisch, heterocyclisch, aliphatisch-heterocylisch, aromatisch, aromatisch-aromatisch oder aliphatisch-aromatisch sein können. In den Resten vorhandene Ringsysteme können ihrerseits mit den genannten Kohlenwasserstoffresten substituiert sein. In offenkettigen Strukturelementen können eine oder mehrere Methylengruppen durch Sauerstoff und/oder Schwefel und/oder NR¹ und/oder NH ersetzt sein und/oder eine oder mehrere CH-Gruppen durch Stickstoff ersetzt sein.

Gegenstand der vorliegenden Erfindung sind auch Phosphininmetallkomplexe, enthaltend ein Metall der 4., 5., 6., 7. oder 8. Nebengruppe des Periodensystems der Elemente und ein oder mehrere Phosphinine der Formeln I, II, III, IV und/oder V. Die Substituenten (R¹-R⁴², Q, X, Y, n, W) dieser Phosphinine besitzen die bereits genannten Bedeutungen.

Bevorzugt sind Phosphininmetallkomplexe, bei denen der Phosphininligand einen Bisarylenrest nach der oben angegebenen, allgemeinen Formel VI als Q-Gruppe enthält.

Im Folgenden werden repräsentative Beispiele von Liganden nach den allgemeinen Formeln I, II, III, IV und V im Sinne dieser Erfindung dargestellt, ohne den Schutzbereich der vorliegenden Erfindung zu beschränken.

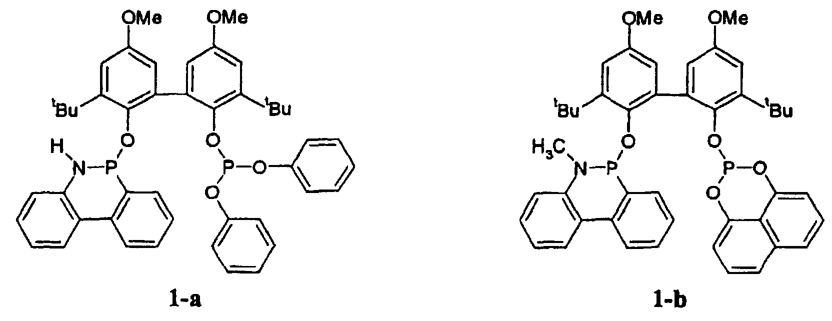

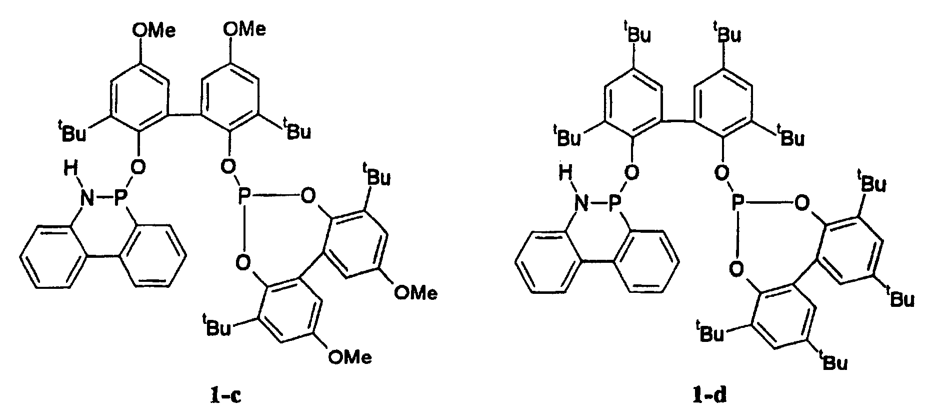

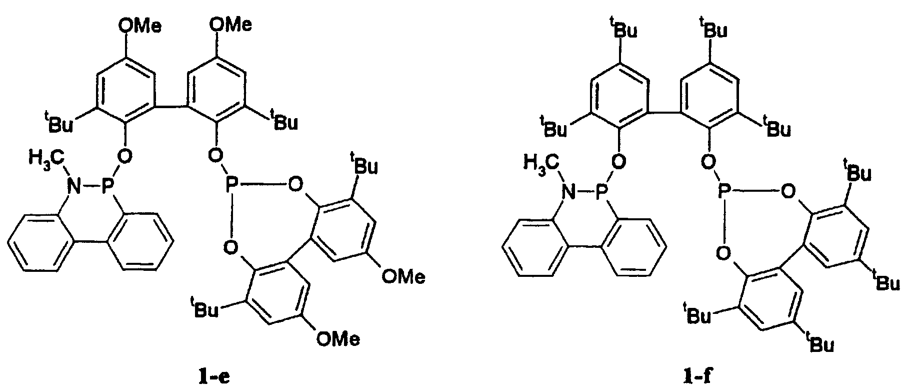

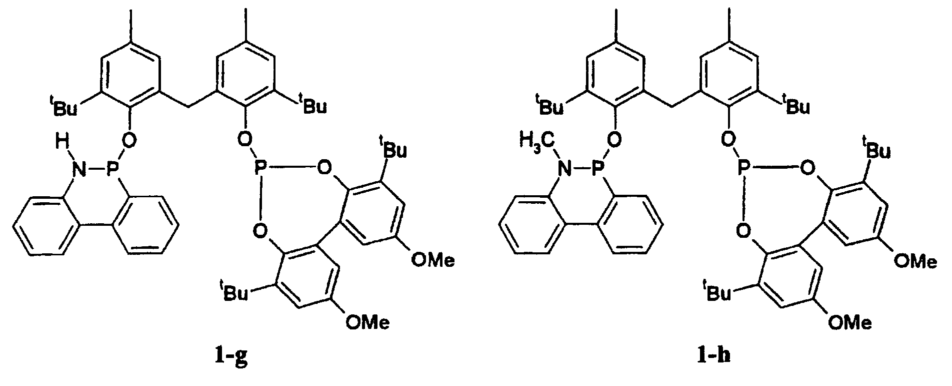

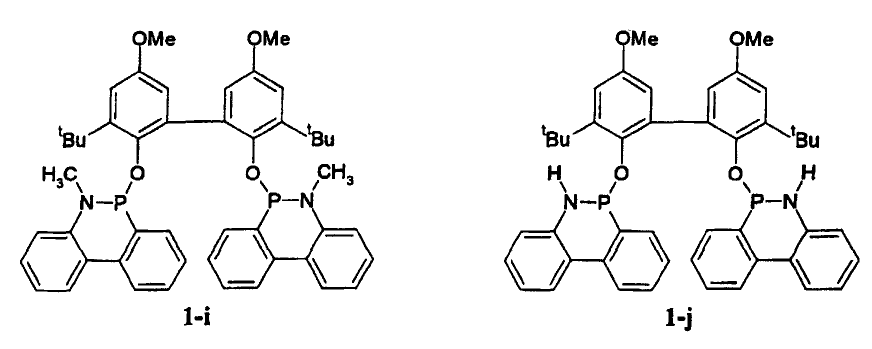

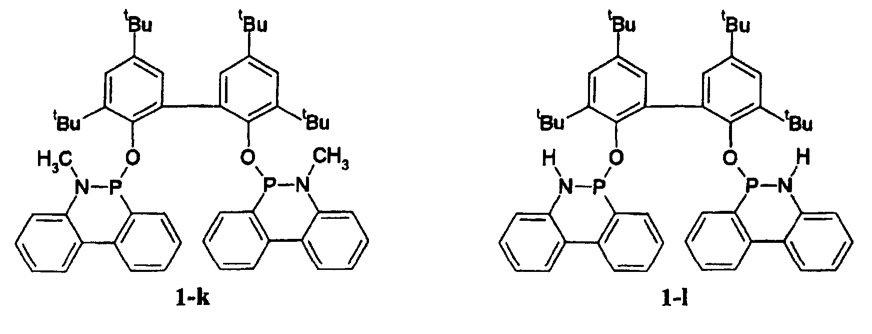

Die erfindungsgemäßen Phosphinine können durch einfache Reaktionen hergestellt werden. Das grundsätzliche Vorgehen wird an einem Weg zu Verbindungen nach der allgemeinen Formel II illustriert
1) Ein Phosphortrihalogenid, vorzugsweise Phosphortrichlorid, wird mit einem Diol oder zwei Moläquivalenten Alkohol zu einem Monohalogenphosphit (Zwischenprodukt A) umgesetzt. 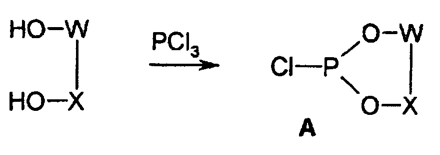
2) Aus dem Zwischenprodukt A wird durch Reaktion mit einem Diol (HO-Q-OH) ein hydroxyl-substituiertes Phosphit erhalten (Zwischenprodukt B). 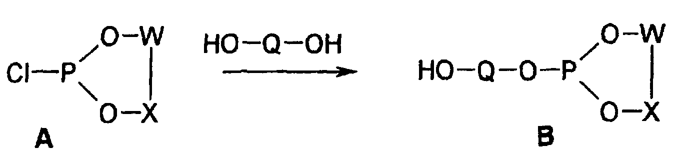
3) Aus der Reaktion von Zwischenprodukt **B** mit der Komponente **C** wird der gewünschte Bidentatligand erhalten.

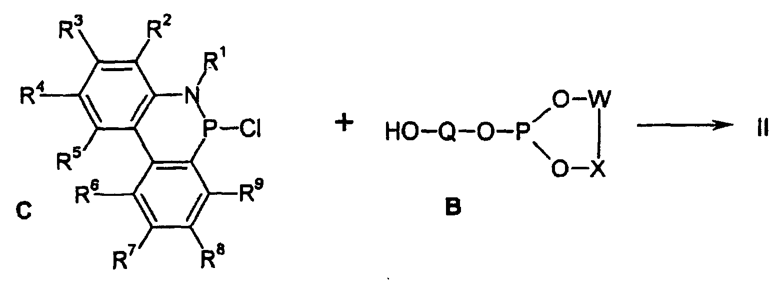

Die Synthese von Verbindungen des Typs **C** ist in der Literatur beschrieben. Sie können zum Beispiel einfach durch Umsetzung von 2-Aminobiphenylen mit Phosphortrichlorid in Gegenwart eines lewissauren Katalysators erhalten werden.

Der Syntheseweg zu Verbindungen nach der allgemeinen Formel **II** ist nur einer von vielen gangbaren, zeigt aber das grundsätzliche Vorgehen. Ein alternativer Weg ist zum Beispiel die Umsetzung von **C** mit der Diolkomponente und anschließende Reaktion mit A zum Zielprodukt.

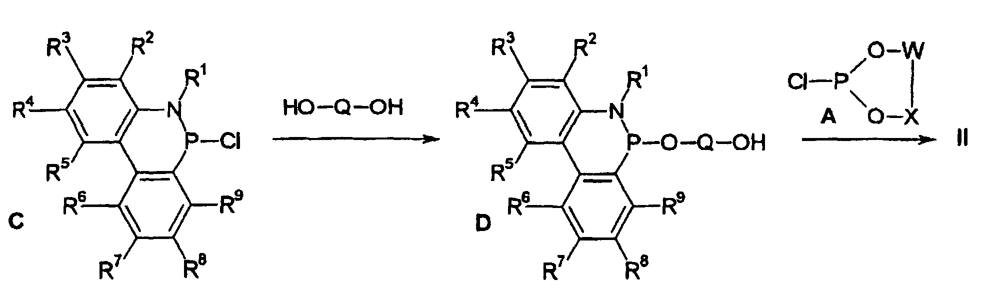

Da die eingesetzten Diole und ihre Folgeprodukte häufig fest sind, werden die Umsetzungen im Allgemeinen in Lösungsmitteln durchgeführt. Als Solventien werden nicht protische Lösungsmittel, die weder mit den Diolen noch mit den Phosphorverbindungen reagieren, verwendet. Geeignete Lösungsmittel sind beispielsweise Tetrahydrofuran, Diethylether oder aromatische Kohlenwasserstoffe wie Toluol.

Bei der Umsetzung von Phosphorhalogeniden mit Alkoholen entsteht Halogenwasserstoff, der durch zugegebene Basen gebunden wird. Beispielsweise werden tertiäre Amine, wie Triethylamin, eingesetzt. Teilweise ist es auch sinnvoll, die Alkohole vor der Reaktion in Metallalkoholate zu überführen, zum Beispiel durch Reaktion mit Natriumhydrid oder Butyllithium.

Die erfindungsgemäßen Phosphinine der Formeln I, II, III, IV und V sind geeignete Bausteine für die Herstellung von Komplexen mit Metallen der 4., 5., 6., 7. oder 8. Nebengruppe des Periodensystems der Elemente. Insbesondere mit Metallen der 8. Nebengruppe können diese Komplexe als Katalysatoren für Carbonylierungsreaktionen oder Hydroformylierungsreaktion verwendet werden, z. B. für die Hydroformylierung von C2-C25-Olefinen. Die Liganden zeichnen sich durch hohe Stabilität aus. Besonders bei Einsatz von Rhodium als Katalysatormetall ergeben sich hohe katalytische Aktivitäten in Hydroformylierungsreaktionen. Aufgrund ihres hohen Molekulargewichtes besitzen die erfindungsgemäßen Phosphinine eine geringe Flüchtigkeit. Sie können daher einfach von den leichter flüchtigen Reaktionsprodukten abgetrennt werden. Sie sind in den gängigen organischen Solventien ausreichend gut löslich.

Weitere Gegenstände der Erfindung sind die Verwendungen der Phosphinine gemäß den Formeln I bis V bzw. der entsprechenden Phosphininmetallkomplexe zur Hydroformylierung von Olefinen, bevorzugt mit 2 bis 25 Kohlenstoffatomen zu den entsprechenden Aldehyden.

Zur Herstellung der katalytisch aktiven Metallkomplexe sind bevorzugt eingesetzte Metalle für die erfindungsgemäßen Phosphinine Rhodium, Kobalt, Platin und Ruthenium. Aus den erfindungsgemäßen Liganden und dem Metall bildet sich unter Reaktionsbedingungen der aktive Katalysator. Die erfindungsgemäßen Liganden können dabei in freier Form in die Reaktionsmischung gegeben werden. Es ist weiterhin möglich, einen Übergangsmetallkomplex, der die o. g. Phosphininliganden enthält, als Precursor für den eigentlichen katalytisch aktiven Komplex einzusetzen. Der Hydroformylierungsprozess kann stöchiometrisch oder mit überschüssigen Mengen (z. B. 1 : 1 bis 1 : 200 Mol-%/Mol-%) an freien Phosphininliganden durchgeführt werden.

Ferner können auch Mischungen verschiedener Liganden - sowohl der erfindungsgemäßen Phosphinine als auch anderer geeigneter phosphorhaltiger Liganden - als freie Ligandkomponente vorhanden sein.

Als zusätzliche, im Reaktionsgemisch vorhandene Liganden können Phosphine, Phosphite, Phosphonite oder Phosphinite eingesetzt werden.

### Beispiele für solche Liganden sind:

Phosphine: Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)-phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin. Phosphite: Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sind sterisch gehinderte Phosphitliganden, wie sie unter anderem in EP 155 508, US 4 668 651, US 4 748 261, US 4 769 498, US 4 774 361, US 4 835 299, US 4 885 401, US 5 059 710, US 5 113 022, US 5 179 055, US 5 260 491, US 5 264 616, US 5 288 918, US 5 360 938, EP 472 071, EP 518 241 und WO 97/20795 beschriebenen werden, geeignete Liganden.
Phosphonite: Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl-, Arylreste oder Halogenatome ersetzt sind und Liganden, die in WO/98 43935, JP 09-268152 und DE 198 10 794 und in den deutschen Patentanmeldungen DE 199 54 721 und DE 199 54 510 beschrieben sind.

Gängige Phosphinitliganden sind unter anderem in US 5 710 344, WO 95/06627, US 5 360 938 oder JP 07082281 beschrieben. Beispiele hierfür sind Diphenyl(phenoxy)phosphin und dessen Derivate, in denen die Wasserstoffatome ganz oder teilweise durch Alkyl-, Arylreste oder Halogenatome ersetzt sind, Diphenyl(methoxy)phosphin, Diphenyl(ethoxy)phosphin.

Im Allgemeinen werden 1 bis 500, vorzugsweise 1 bis 200, bevorzugt 3 bis 50 Mol des erfindungsgemäßen Liganden pro Mol Gruppe-VIII-Übergangsmetall eingesetzt. Frischer Ligand kann zu jedem Zeitprodukt der Reaktion zugesetzt werden, um die Konzentration an freiem Liganden konstant zu halten. Die erfindungsgemäßen Übergangsmetall-Phosphininkomplex-Katalysatoren können vor ihrem Einsatz synthetisiert werden. In der Regel werden aber die katalytisch aktiven Komplexe aus einem Katalysatorvorläufer und dem erfindungsgemäßen Phosphininliganden in situ im Reaktionsmedium gebildet.

Als Katalysatorvorläufer kommen Salze oder Komplexe der Übergangsmetalle zum Einsatz. Beispiele sind Rhodiumcarbonyle, Rhodiumnitrat, Rhodiumchlorid, Rh(CO)₂(acac) (acac = Acetylacetonat), Rhodiumacetat, Rhodiumoctanoat oder Rhodiumnonanoat.

Die Konzentration des Metalls im Reaktionsgemisch liegt im Bereich von 1 ppm bis 1000 ppm, vorzugsweise im Bereich von 5 ppm bis 300 ppm.

Die mit den erfindungsgemäßen Phosphininen bzw. den entsprechenden Metallkomplexen durchgeführte Hydroformylierungsreaktion erfolgt nach bekannten Vorschriften, wie z. B. in J. FALBE, "New Syntheses with Carbon Monoxide", Springer Verlag, Berlin, Heidelberg, New York, Seite 95 ff., (1980) beschrieben.

Die Reaktionstemperaturen für ein Hydroformylierungsverfahren mit den erfindungsgemäßen Phosphininen bzw. Phosphininmetallkomplexen als Katalysator liegen zwischen 40 °C und 180 °C, vorzugsweise zwischen 75 °C und 140 °C. Die Drücke, unter denen die Hydroformylierung abläuft, betragen 1-300 bar Synthesegas, vorzugsweise 15-64 bar. Das Molverhältnis zwischen Wasserstoff und Kohlenmonoxid (H₂/CO) im Synthesegas beträgt 10/1 bis 1/10, bevorzugt 1/1 bis 2/1.

Der Katalysator bzw. der Ligand ist homogen im Hydroformylierungsgemisch, bestehend aus Edukt (Olefine) und Produkten (Aldehyden, Alkoholen, im Prozess gebildete Hochsieder), gelöst. Optional kann zusätzlich ein Lösungsmittel verwendet werden.

Die Edukte für die Hydroformylierung sind Monoolefine oder Gemische von Monoolefinen mit 2 bis 25 Kohlenstoffatomen mit end- oder innenständiger C-C-Doppelbindung. Sie können geradkettig, verzweigt oder von cyclischer Struktur sein und auch mehrere olefinisch ungesättigte Gruppen aufweisen. Beispiele sind Propen, 1-Buten, c-2-Buten, t-2-Buten, Isobuten, Butadien, Mischungen der C4-Olefine, 1- oder 2-Penten, 2-Methylbuten-1, 2-Methylbuten-2, 3-Methylbuten-1, 1-, 2-oder 3-Hexen, das bei der Dimerisierung von Propen anfallende C6-Olefingemisch (Dipropen), 1-Hepten, Heptene, 2- oder 3-Methyl-1-hexen, 1-Octen, Octene, 2-Methylheptene, 3-Methylheptene, 5-Methylhepten-2, 6-Methylhepten-2, 2-Ethylhexen-1, das bei der Dimerisierung von Butenen anfallende isomere C8-Olefingemisch (Dibuten), 1-Nonen, Nonene, 2- oder 3-Methyloctene, das bei der Trimerisierung von Propen anfallende C9-Olefingemisch (Tripropen), Decene, 2-Ethyl-1-octen, Dodecene, das bei der Tetramerisierung von Propen oder der Trimerisierung von Butenen anfallende C12-Olefingemisch (Tetrapropen oder Tributen), Tetradecene, Hexadecene, bei der Tetramerisierung von Butenen anfallende C16-Olefingemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher C-Zahl. Ebenfalls können Olefine oder Olefingemische, die durch Fischer-Tropsch-Synthese erzeugt werden, eingesetzt werden, sowie Olefine, die durch Oligomerisierung von Ethen erhalten werden oder die über Methathesereaktionen oder Telomerisationsreaktion zugänglich sind.

Bevorzugte Edukte sind Propen, 1-Buten, 2-Buten, 1-Hexen, 1-Octen, Dimere und Trimere des Butens (Dibuten, Di-n-buten, Di-iso-buten, Tributen) und allgemein α-Olefine.

Die Hydroformierung kann kontinuierlich oder diskontinuierlich durchgeführt werden. Beispiele für technische Ausführungen sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen vom Katalysator kann durch eine herkömmliche Methode, wie Fraktionierung, durchgeführt werden. Technisch kann dies beispielsweise über eine Destillation, über einen Fallfilmverdampfer oder einen Dünnschichtverdampfer erfolgen. Die gilt besonders, wenn der Katalysator in einem hochsiedenden Lösungsmittel gelöst von den niedriger siedenden Produkten abgetrennt wird. Die abgetrennte Katalysatorlösung kann für weitere Hydroformylierungen verwendet werden. Bei Einsatz niederer Olefine (z. B. Propen, Buten, Penten) ist auch ein Austrag der Produkte aus dem Reaktor über die Gasphase möglich.

Die folgenden Beispiele sollen die Erfindung erläutern, nicht aber ihren Anwendungsbereich beschränken, der sich aus den Patentansprüchen ergibt.

### Beispiele

Alle Präparationen wurden mit Standard-Schlenk-Technik unter Schutzgas durchgeführt. Die Lösungsmittel wurden vor Gebrauch über geeigneten Trocknungsmitteln getrocknet.

### Beispiel 1

### Darstellung von 10-Chloro-9-Methyl-9, 10-dihydro-9-aza-10-phosphaphenanthren

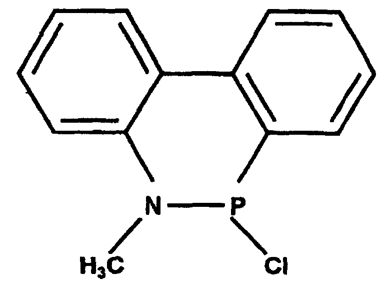

a) 2-Ethoxycarbonylaminobiphenyl
   Zu einer Mischung von 25 g 2-Aminobiphenyl (0.148 Mol) und 79 g Pyridin (1 Mol) in 150 ml Dichlormethan wird langsam unter Rühren eine Lösung von 21.7 g (0.2 Mol) Chlorameisensäureethylester in 20 ml Dichlormethan getropft. Nach 12 h Rühren erfolgt Zugabe von 100 ml 10 %-iger NaOH. Die wässrige Phase wird abgetrennt und mit Dichlormethan ausgeschüttelt. Nach Trocknen über Natriumsulfat wird das Lösungsmittel im Vakuum entfernt und das zurückbleibende rote Öl säulenchromatografisch (Kieselgel 60, 0.043...0.060 mm, Eluent Hexan/Ethylacetat = 9:1) aufgearbeitet. Ausbeute: 31.7 g = 89 %.
b) *N*-Methyl-*o*-phenylanilin
   Zu einer Suspension von 14.2 g (0.373 Mol) Lithiumaluminiumhydrid in 300 ml THF wird eine Lösung von 18 g (0.075 Mol) 2-Ethoxycarbonylaminobiphenyl in 100 ml THF getropft. Es wird 1 h nachgerührt und dann 3 h am Rückfluss erhitzt. Anschließend setzt man unter Eiskühlung zunächst tropfenweise 28 ml Wasser, dann 18 ml 15 %-ige Natronlauge zu. Nach Abtrennen des Niederschlages wird dieser mehrmals mit Ether gewaschen. Die vereinigten Filtrate werden mit Natriumsulfat getrocknet, und die nach Verdampfen des Lösungsmittels erhaltene rötliche Flüssigkeit bei 0.01 mbar fraktioniert. Kp.: 65-67 °C.
   Ausbeute: 10.64 g = 77 %.
c) 10-chloro-9-methyl-9,10-dihydro-9-aza-10-phospha-phenanthren (Vorstufe C1)
   Die Zyklisierung der Aminoverbindung mit PCl₃, erfolgt in Analogie zur Vorschrift für das 9-H-Derivat: M. J. S. Dewar, V. P. Kubba, *J. Amer.Chem.Soc*. 1960, 82, 5685-5688. Ausgehend von 10 g (0.052 Mol) *N*-Methylaminobiphenyl werden 9 g = 70 % der Chlorverbindung erhalten. ³¹P-NMR: (C₆D₆) δ 99.09 ppm.

### Beispiel 2

### Synthese der Vorstufe B

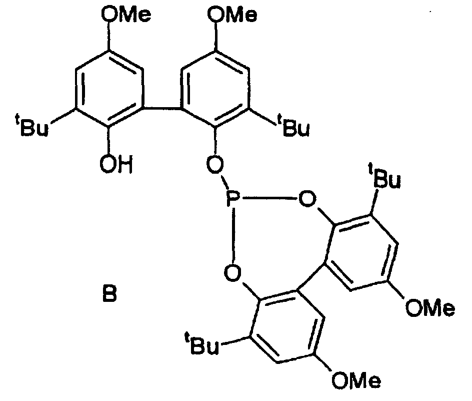

### Vorstufe B

Zu einer Lösung von 2.42 g 2,2'-Bis(6-*tert*.-butyl-1-hydroxy-4-methoxyphenyl) (6,75 mmol) und 1.6 ml Pyridin in 22 ml THF tropft man bei 0 °C eine Lösung von 0.93 g PCl₃ (6.75 mmol) in 10 ml THF. Nach 4 h Rühren bei 25 °C wird das Lösungsmittel im Vakuum entfernt. Nach Zusatz von 40 ml Diethylether, Filtration und Einengen im Vakuum werden 2.8 g (98 %) an spektroskopisch reinem Chlorophosphorigsäureester des 2,2'-Bis(6-*tert*.-butyl-1-hydroxy-4-methoxyphenyl) erhalten: ³¹P-NMR (CD₂Cl₂) δ 172.7 ppm. 2.8 g dieses Chloroesters (6.62 mmol) in 20 ml THF gibt man bei Raumtemperatur zu einer bei -20 °C erhaltenen Monolithiumphenolatlösung aus 2.37 g 2,2'-Bis(6-*tert*.-butyl-1-hydroxy-4-methoxyphenyl) (6.62 mmol) in 30 ml THF und 20.7 ml einer 0.32 *M* Hexanlösung von n-Butyllithium (6.62 mmol). Nach 24 h wird im Vakuum eingeengt. Zugabe von 40 ml Methylenchlorid, Filtration und Entfernen des Solvens im Vakuum ergeben 4,6 g (93 %) an hochviskosem Produkt.
Analyse (ber. für C₄₄H₅₇O₈P = 744.9 g/ Mol) C 70.35 (70.95); H 7.86 (7.71 ). ³¹P-NMR (CD₂Cl₂) δ 140.7 ppm. ¹H-NMR (CD₂Cl₂) δ 1.43 (s, 9 H); 1.56 (s, 9 H); 1.63 (s, 9 H); 1.67 (s, 9 H); 4.01 (s, 3 H);4.03 (s, 6 H); 4.05 (s, 3 H); 5.42 (s, 1 H); 6.7...7.3 (m, 8 H) ppm. FAB MS: *m*/*e* 745 (37%, *M*⁺); 387 (100 %, *M*⁺- 2,2'-Bis(6-*tert*.-butyl-1-hydroxy-4-methoxyphenyl)). IR (CHCl₃, 0.1 mm CaF₂), ν (OH) = 3549 cm⁻¹.

### Beispiel 3

### Synthese eines Phosphinins gemäß 1-c

Zu 3.06 g Vorstufe B aus Beispiel 2 (4.11 mmol) in 32 ml THF wird bei -20 °C unter Rühren ein Äquivalent n-Butyllithium, gelöst in 13 ml Hexan, getropft. Nach Erwärmen auf Raumtemperatur und 30 min Rühren wird die erhaltene Lösung zu 0.961 g 10-chloro-9,10-dihydro-9-aza-10-phospha-phenanthren (Vorstufe C-2 aus Beispiel 4) (4.11 mmol), gelöst in 7 ml THF, gegeben und die Mischung 4 h bei Raumtemperatur gerührt. Der nach Abziehen des Lösungsmittelgemisches im Vakuum erhaltene Rückstand wird in 80 ml Hexan verrieben. Man filtriert und extrahiert den Frittenrückstand durch 5-malige Destillation aus der Mutterlauge. Einengen der Lösung auf 50 % des Ausgangsvolumens und 3 Tage Lagerung bei -28 °C ergibt das Rohprodukt, welches nochmals aus heißem Hexan umkristallisiert wird. Ausbeute: 1.9 g = 48 %.
Analyse (ber. auf C₅₆H₆₅O₈P₂N = 942.07 g/mol): C 71.50 (71.40); H 7.25 (6.95); N 1.56 (1.47) %. ³¹P-NMR (CD₂Cl₂): δ 82.55; 87.1; 139.0; 142.7.
Diastereomerenverhältnis = 3.3 : 1. ¹H-NMR (CD₂Cl₂): δ 0.91..1.44 (36 H); 3.74..3.85 (12 H), 4.88..5.08 (1 H); 6.51..8.1 (16 H). FAB-MS: *m*/*e* 942 (7 %, *M*⁺); 746 (26 %); 539 (21 %); 388 (37 %); 199 (100 %).

### Beispiel 4

### Darstellung von 10-Chloro-9,10-dihydro-9-aza-10-phosphaphenanthren

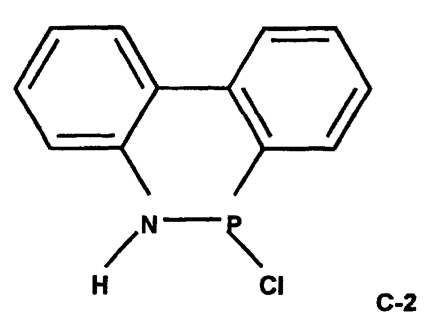

Diese Verbindung wurde gemäß M. J. S. Dewar, V. P. Kubba, *J. Amer. Chem. Soc. 1960, 82, 5685-5688* (*S. 20, Z. 19)* hergestellt und wird im Folgenden als "C-2" bezeichnet.

### Beispiel 5

### Synthese eines Phosphins gemäß 1-e

Zu 1.85 g Vorstufe B aus Beispiel 2 (2.49 mmol) in 20 ml THF wird bei -20 °C unter Rühren ein Äquivalent n-Butyllithium, gelöst in 8 ml Hexan, getropft. Nach Erwärmen auf Raumtemperatur und 30 min Rühren wird die erhaltene Lösung zu 0.616 g 10-Chloro-9-methyl-9,10-dihydro-9-aza-10-phospha-phenanthren (Vorstufe C-1 aus Beispiel 1) (2.49 mmol), gelöst in 5 ml THF, gegeben und die Mischung 4 h bei Raumtemperatur gerührt. Der nach Abziehen des Lösungsmittelgemisches im Vakuum erhaltene Rückstand wird in 40 ml Hexan verrieben. Man filtriert und extrahiert den Frittenrückstand durch 5-malige Rückdestillation aus der Mutterlauge. Mehrtägiges Stehenlassen bei Raumtemperatur ergibt einen weißen Niederschlag, der filtriert, mit 10 ml Hexan gewaschen und bei 70 °C Badtemperatur im Vakuum getrocknet wird. Ausbeute: 1.99 g = 84 %. Analyse (ber. für C₅₇H₆₇O₈P₂N = 956 g/mol): C 72.02 (71.61); H 7.69 (7.06); P 6.48 (5.99); N 1.39 (1.46). ³¹P-NMR (CD₂Cl₂): δ 101.2, 102.2; 141.7; 144.7. Diastereomerenverhältnis = 7.5:1. ¹H-NMR (CD₂Cl₂): δ 0.9..1.45 (36 H); 3.18..3.43 (3 H); 3.74..3.94 (12 H), 6.14..8.15 (16 H). Cl-MS: *m*/*e* 956 (10 %, *M*⁺); 745 (100 %)

### Beispiele 6 und 7

### Hydroformylierung von 1-Octen

Die Versuchsdurchführung erfolgte nach Befüllen unter Schutzgas in einem mit Begasungsrührer, Druckpipette und Nachdruckregler ausgestatteten 200 ml-Edelstahlautoklav der Fa. Buddeberg, Mannheim, im Ölbadthermostaten. Zur Minimierung eines Einflusses von Feuchtigkeit und Sauerstoff wurde das als Solvens benutzte Toluol mit Natrium-Ketyl getrocknet und unter Argon destilliert. Das als Substrat eingesetzte 1-Octen wurde mehrere Stunden über Natrium am Rückfluß erhitzt und unter Argon destilliert.
Der Autoklav wurde beschickt mit 27 ml Toluol, in welchem 5.456 mg= 0.0176 mmol [acacRh(COD)], und 0.088 mmol des jeweiligen Liganden gelöst waren. Das Verhältnis Rh/P betrug damit 1:10. In die Druckpipette über dem Reaktor gab man 24 ml = ca. 16.8 g (149.3 mmol) 1-Octen. Das Verhältnis Rh/1-Octen betrug damit ca. 1: 8500. Reaktor und Druckpipette wurden über einen der Druckregelstrecke parallel geschalteten Bypass bei einem Solldruck von 50 bar mit 33 bar, bei einem Solldruck von 20 bar mit 13 bar CO/H₂ (1:1; Synthesegas) beaufschlagt und der Reaktorinhalt unter magnetischem Rühren mit dem Begasungsrührer mit 1500 min⁻¹ auf 100°C temperiert. Nach Erreichen der Solltemperatur wurde der Druck auf 47 bar (17 bar) erhöht und das Olefingemisch aus der Druckpipette mit einem Druck von 55 bar (25 bar) in den Reaktor gepreßt. Es stellte sich ein Anfangsdruck der Reaktion von 49.6 bar (19.2 bar) ein. Nach sofortiger manueller Regulierung auf 50 bar (20 bar) wurde der Bypass geschlossen, und der Druck über die gesamte Reaktionszeit mit dem Nachdruckregler konstant gehalten. Der Versuch wurde unter Zwangskühlung nach Ablauf der festgelegten Reaktionszeit beendet. Die Reaktionslösung wurde unter Schutzgas entnommen und gaschromatographisch analysiert.
Die nachfolgende Tabelle enthält die mit den einzelnen Liganden erhaltenen Ergebnisse.

| **Beispiel** | **Ligand** | **Temp. [°C]** | **p [bar]** | **t [h]** | **Ausbeute [%]** | **Nonanal Anteil [%]** |
|---|---|---|---|---|---|---|
| 6 | 1-c | 100 | 50 | 3 | 70 | 94.3 |
| 7 | 1-e | 100 | 20 | 3 | 49 | 93.6 |

### Beispiele 8 und 9

### Hydroformylierung einer Mischung von 1-Octen, 2-Octen, 3-Octen und 4-Octen

Die Versuchsdurchführung erfolgte nach Befüllen unter Schutzgas in einem mit Begasungsrührer, Druckpipette und Nachdruckregler ausgestatteten 200 ml-Edelstahlautoklav der Fa. Buddeberg, Mannheim, im Ölbadthermostaten. Zur Minimierung eines Einflusses von Feuchtigkeit und Sauerstoff wurde das als Solvens benutzte Toluol mit Natrium-Ketyl getrocknet und unter Argon destilliert. Das als Substrat eingesetzte Octenisomerengemisch wurde mehrere Stunden über Natrium am Rückfluß erhitzt und unter Argon destilliert. Zusammensetzung: 1-Octen, 3.3 %; *cis*+*trans*-2-Octen, 48.5 %; *cis*+*trans*-3-Octen, 29.2%; *cis*+*trans*-Octen-4, 16.4 %; verzweigte C8-Olefine, 2.6 %.
Der Autoklav wurde beschickt mit 41 ml Toluol, in welchem 18.75 mg= 0.0604 mmol [acacRh(COD)], der jeweilige Bidentatligand und der nachfolgend abgebildete Coliganden CL1 gelöst waren. Das Verhältnis Rh/Bidentatligand (Ligand)/Etherphosphonit (Coligand) ist in der Tabelle angegeben. In die Druckpipette über dem Reaktor gab man 15 ml = 10.62 g (94.63 mmol) Octene. Das Verhältnis Rh/Octene betrug damit ca. 1: 1570. Reaktor und Druckpipette wurden über einen der Druckregelstrecke parallel geschalteten Bypass mit 13 bar CO/H₂ (1:1; Synthesegas) beaufschlagt und der Reaktorinhalt unter magnetischem Rühren mit dem Begasungsrührer mit 1500 min⁻¹ auf 130 °C temperiert. Nach Erreichen der Solltemperatur wurde der Druck auf 17 bar erhöht, und das Olefingemisch aus der Druckpipette mit einem Druck von 25 bar in den Reaktor gepreßt. Es stellte sich ein Anfangsdruck der Reaktion von 19.2 bar ein. Nach sofortiger manueller Regulierung auf 20 bar wurde der Bypass geschlossen, und der Druck über die gesamte Reaktionszeit mit dem Nachdruckregler konstant gehalten. Der Versuch wurde unter Zwangskühlung nach drei Stunden beendet. Die Reaktionslösung wurde unter Schutzgas entnommen und gaschromatographisch analysiert.
Die nachfolgende Tabelle enthält die mit den einzelnen Liganden erhaltenen Ergebnisse.

| **Beispiel** | **Ligand / Coligand** | **T [°C]** | **Rh/Lig/CoLig/Olefin [mol/mol/mol/mol]** | **t [h]** | **Ausb. [%]** | **Nonanal Anteil [%]** |
|---|---|---|---|---|---|---|
| 8 | 1-c/ CL-1 | 130 | 1/2.5/5/1570 | 6 | 36 | 75.6 |
| 9 | 1-e / CL-1 | 130 | 1/ 2.5/5/1570 | 6 | 81 | 33,6 |

### Eingesetzter Coligand

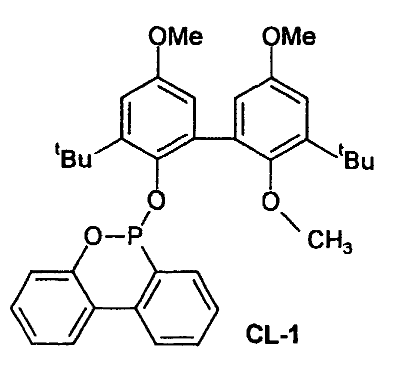

## Patentansprüche

1. Phosphinin der Formel I 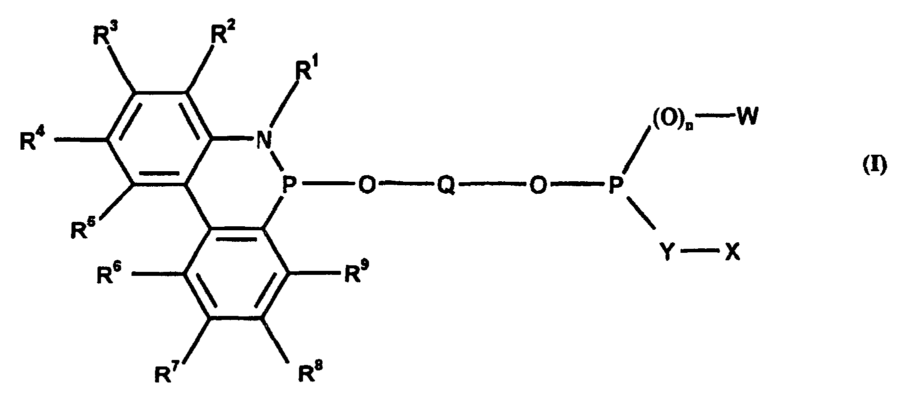 mit
n = 0 oder 1
Y = O, NH, NR¹
R¹ = H, aliphatischer oder aromatischer Kohlenwasserstoffrest mit 1 bis 25 Kohlenstoffatomen
R², R³, R⁴, R⁵, R⁶, R⁷, R⁸, R⁹ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R² bis R⁹ eine gleiche oder unterschiedliche Bedeutung besitzen und kovalent miteinander verknüpft sein können,
F, Cl, Br, I, -CF₃, -OR¹⁰, -COR¹⁰, -CO₂R¹⁰, -CO₂M, -SR¹⁰, -SO₂R¹⁰, -SOR¹⁰, -SO₃R¹⁰, -SO₃M, -SO₂NR¹⁰R¹¹, NR¹⁰R¹¹, N=CR¹⁰R¹¹, NH₂,
R¹⁰, R¹¹ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, die gleich oder unterschiedlich oder kovalent miteinander verknüpft sein können.

2. Phosphinin gemäß Anspruch 1,
**dadurch gekennzeichnet,**
**dass** W und X aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, mit einer kovalenten Verknüpfung gemäß Formel II 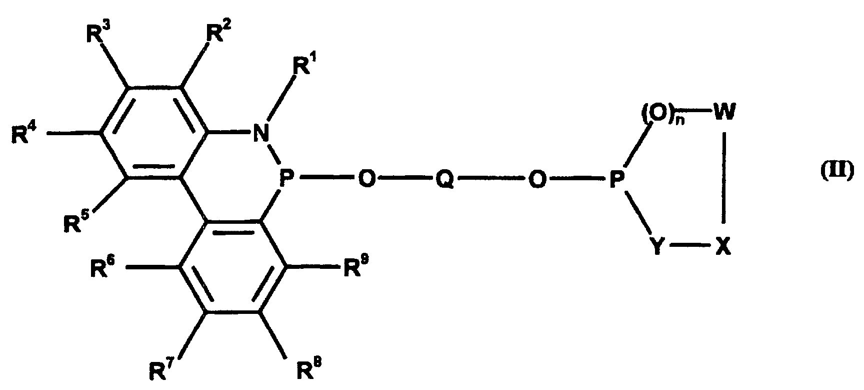 sind und R¹, R², R³, R⁴, R⁵, R⁶, R⁷, R⁸, R⁹, W, X, Y, n und Q die in Anspruch 1 genannten Bedeutungen besitzen.

3. Phosphinin gemäß Anspruch 1,
**dadurch gekennzeichnet,**
**dass** W und X aromatische Kohlenwasserstoffreste mit 1 bis 50 Kohlenstoffatomen mit kovalenten Verknüpfungen gemäß Formel III 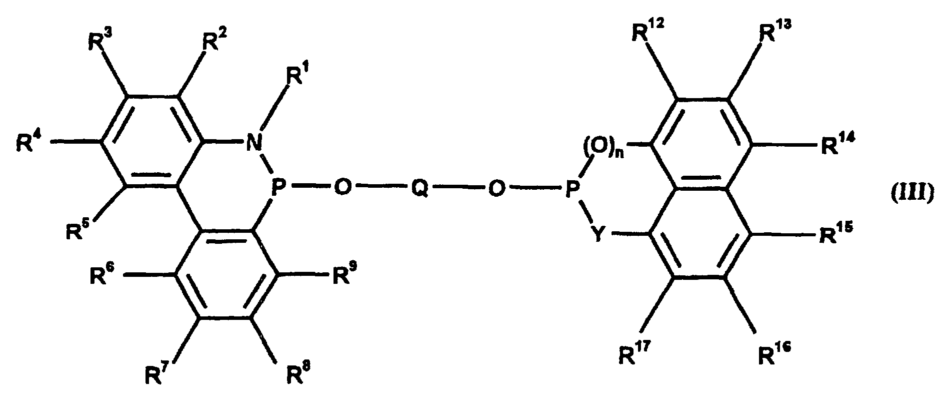 sind,
mit R¹², R¹³, R¹⁴, R¹⁵, R¹⁶, R¹⁷ = H, aliphatischer, alicyclischer, äliphatischalicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R¹² bis R¹⁷ eine gleiche oder unterschiedliche Bedeutung besitzen und kovalent miteinander verknüpft sein können,
F, Cl, Br, I, -CF₃, -OR¹⁸, -COR¹⁸, -CO₂R¹⁸, -CO₂M, -SR¹⁸, -SO₂R¹⁸, -SOR¹⁸, - SO₃R¹⁸, -SO₃M, -SO₂NR¹⁸R¹⁹, NR¹⁸R¹⁹, N=CR¹⁸R¹⁹, NH₂,
R¹⁸, R¹⁹ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴, R⁵, R⁶, R⁷, R⁸, R⁹, n, Y und Q die in Anspruch 1 genannten Bedeutungen besitzen.

4. Phosphinin nach einem der Ansprüche 1 oder 2,
**dadurch gekennzeichnet,**
**dass** W und X aromatische Kohlenwasserstoffreste mit 1 bis 50 Kohlenstoffatomen mit einer kovalenten Verknüpfung gemäß Formel IV sind 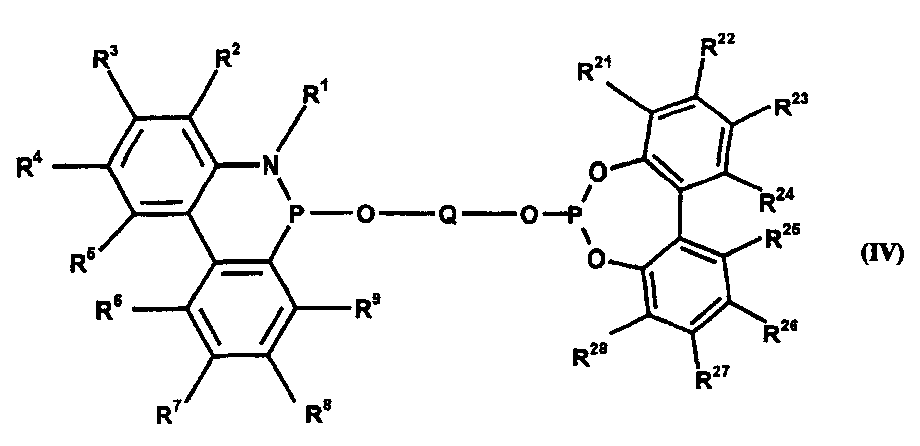 mit
R²¹, R²², R²³, R²⁴, R²⁵, R²⁶, R²⁷, R²⁸ für H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R²¹ bis R²⁸ eine gleiche oder unterschiedliche Bedeutung besitzen und kovalent miteinander verknüpft sein können,
F, Cl, Br, I, -CF₃, -OR²⁹, -COR²⁹, -CO₂R²⁹, -CO₂M, -SR²⁹, -SO₂R²⁹, -SOR²⁹, - SO₃R²⁹, -SO₃M, -SO₂NR²⁹R³⁰, NR²⁹R³⁰, N=CR²⁹R³⁰, NH₂,
R²⁹, R³⁰ = H, substituierter oder unsubstituierter, aliphatischer oder aromatischer Kohlenwasserstoffrest mit 1 bis 25 Kohlenstoffatomen,
M = Alkalimetall, Erdalkalimetall, Ammonium, Phosphoniumion,
wobei
R¹, R², R³, R⁴, R⁵, R⁶, R⁷, R⁸, R⁹ und Q die in Anspruch 1 genannten Bedeutungen besitzen.

5. Phosphinin nach einem der Ansprüche 1 bis 2,
**dadurch gekennzeichnet,**
**dass** W und X aromatische Kohlenwasserstoffreste mit 1 bis 50 Kohlenstoffatomen mit kovalenten Verknüpfungen gemäß Formel V 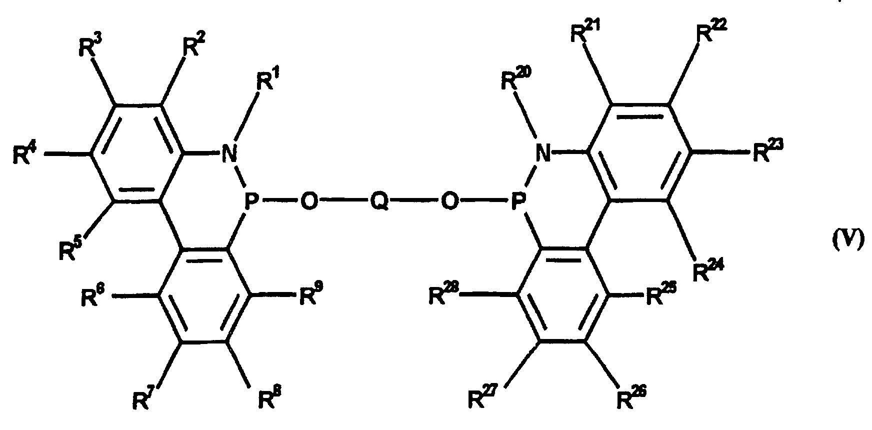 sind,
mit
R²⁰ = H, aliphatischer oder aromatischer Kohlenwasserstoffrest mit 1 bis 25 Kohlenstoffatomen,
R²¹, R²², R²³, R²⁴, R²⁵, R²⁶, R²⁷, R²⁸ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R²¹ bis R²⁸ eine gleiche oder unterschiedliche Bedeutung besitzen und kovalent miteinander verknüpft sein können,
F, Cl, Br, I, -CF₃, -OR²⁹, -COR²⁹, -CO₂R²⁹, -CO₂M, -SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, substituierter oder unsubstituierter, aliphatischer oder aromatischer Kohlenwasserstoffrest mit 1 bis 25 Kohlenstoffatomen,
M = Alkalimetall, Erdalkalimetall, Ammonium, Phosphoniumion
und
R¹, R², R³, R⁴, R⁵, R⁶, R⁷, R⁸, R⁹ und Q die in Anspruch 1 genannten Bedeutungen besitzen, wobei R² bis R⁹ eine gleiche oder unterschiedliche Bedeutung besitzen und kovalent miteinander verknüpft sein können.

6. Phosphinin nach einem der Ansprüche 1 bis 5,
**dadurch gekennzeichnet,**
**dass** das Q ein Kohlenwasserstoffrest gemäß Formel VI ist 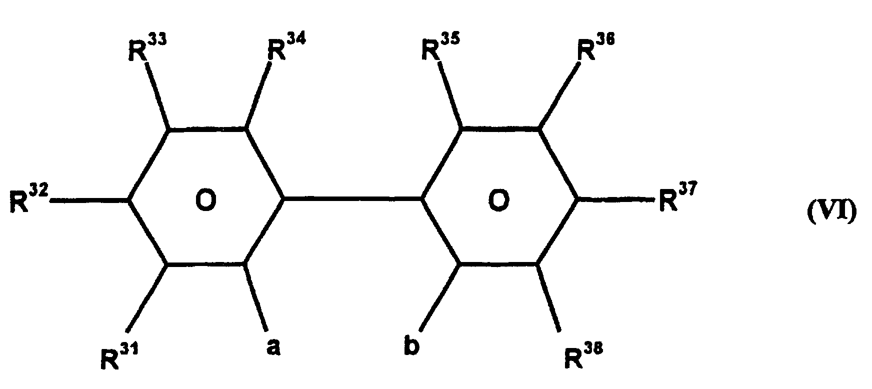 mit
R³¹, R³², R³³, R³⁴, R³⁵, R³⁶, R³⁷, R³⁸ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR³⁹, -COR³⁹, -CO₂R³⁹, -CO₂M, - SR³⁹, -SO₂R³⁹, -SOR³⁹, -SO₃R³⁹, -SO₃M, -SO₂NR³⁹R⁴⁰, NR³⁹R⁴⁰, N=CR³⁹R⁴⁰, NH₂,
R³⁹, R⁴⁰ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion
wobei die Positionen a und b als Anknüpfpunkte dienen.

7. Phosphininmetallkomplex, enthaltend ein Metall der 4., 5., 6., 7. oder 8. Nebengruppe des Periodensystems der Elemente und ein oder mehrere Phosphinine der Formel I 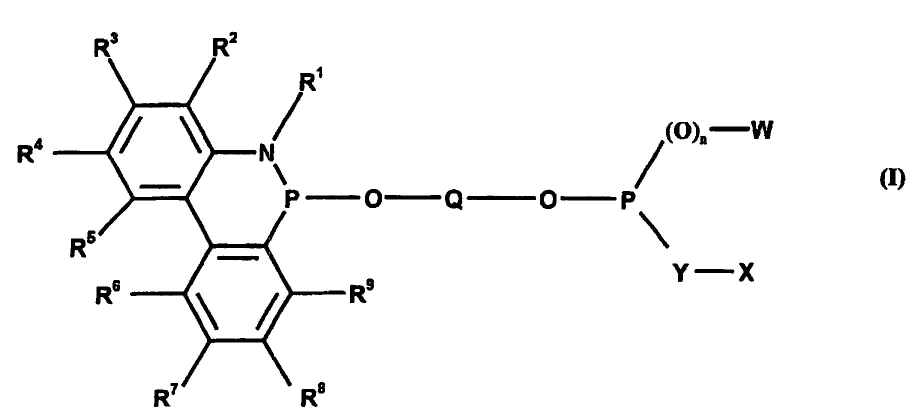 mit
n = 0 oder 1
Y = O, NH, NR¹
R¹ = H, aliphatischer oder aromatischer Kohlenwasserstoffrest mit 1 bis 25 Kohlenstoffatomen
R², R³, R⁴, R⁵, R⁶, R⁷, R⁸, R⁹ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R² bis R⁹ eine gleiche oder unterschiedliche Bedeutung besitzen und kovalent miteinander verknüpft sein können,
F, Cl, Br, I, -CF³, -OR¹⁰, -COR¹⁰, -CO₂R¹⁰, -CO₂M, -SR¹⁰, -SO₂R¹⁰, -SOR¹⁰, -SO₃R¹⁰, -SO₃M, -SO₂NR¹⁰R¹¹, NR¹⁰R¹¹, N=CR¹⁰R¹¹, NH₂, R¹⁰, R¹¹ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion
Q = zweiwertiger aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen,
W, X = aliphatische, alicyclische, aliphatisch-alicyclische, heterocyclische, aliphatisch-heterocyclische, aromatische, aromatisch-aromatische, aliphatisch-aromatische Kohlenwasserstoffreste mit 1 bis 50 Kohlenstoffatomen, die gleich oder unterschiedlich oder kovalent miteinander verknüpft sein können.

8. Phosphininmetallkomplex nach Anspruch 7,
**dadurch gekennzeichnet,**
**dass** W und X aliphatische, alicyclische, aliphatisch-alicyclische, heterocyclische, aliphatisch-heterocyclische, aromatische, aliphatisch-aromatische Kohlenwasserstoffreste mit 1 bis 50 Kohlenstoffatomen, mit einer kovalenten Verknüpfung gemäß Formel II 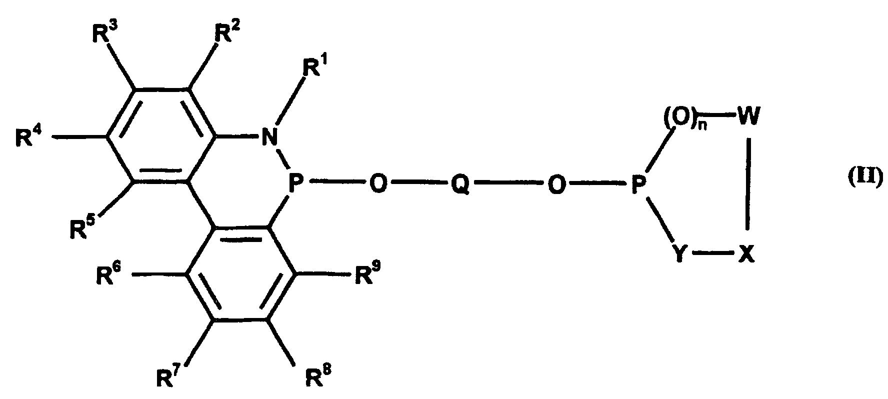 sind und R¹, R², R³, R⁴, R⁵, R⁶, R⁷, R⁸, R⁹, W, X, Y, n und Q die in Anspruch 1 genannten Bedeutungen besitzen.

9. Phosphininmetallkomplex nach Anspruch 7,
**dadurch gekennzeichnet,**
**dass** W und X aromatische Kohlenwasserstoffreste mit 1 bis 50 Kohlenstoffatomen mit kovalenten Verknüpfungen gemäß Formel III 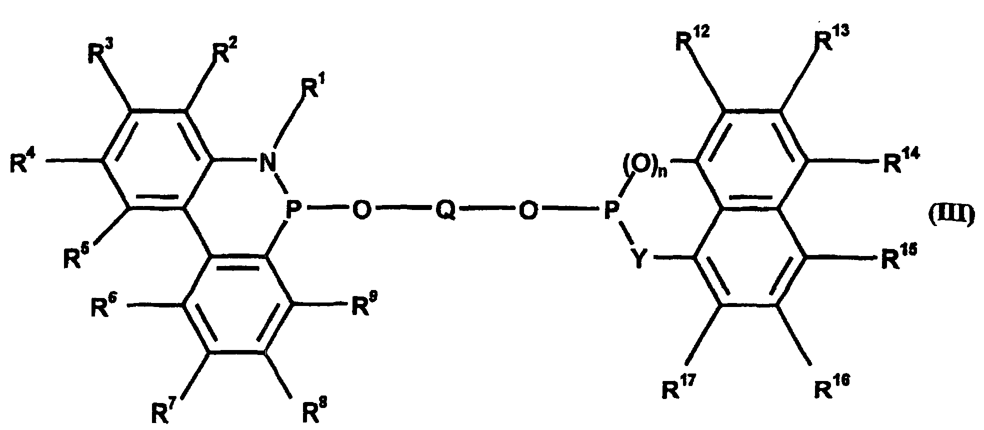 sind,
mit R¹², R¹³, R¹⁴, R¹⁵, R¹⁶, R¹⁷ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R¹² bis R¹⁷ eine gleiche oder unterschiedliche Bedeutung besitzen und kovalent miteinander verknüpft sein können,
F, Cl, Br, I, -CF₃, -OR¹⁸, -COR¹⁸, -CO₂R¹⁸, -CO₂M, -SR¹⁸, -SO₂R¹⁸, -SOR¹⁸, -SO₃R¹⁸, -SO₃M, -SO₂NR¹⁸R¹⁹, NR¹⁸R¹⁹, N=CR¹⁸R¹⁹, NH₂, R¹⁸, R¹⁹ = H, substituierter oder unsubstituierter, aliphatischer oder aromatischer Kohlenwasserstoffrest mit 1 bis 25 Kohlenstoffatomen, mit gleicher oder unterschiedlicher Bedeutung,
M = Alkalimetall-, Erdalkalimetall-, Ammonium-, Phosphoniumion und
R¹, R², R³, R⁴, R⁵, R⁶, R⁷, R⁸, R⁹, n, Y und Q die in Anspruch 7 genannten Bedeutungen besitzen.

10. Phosphininmetallkomplex nach einem der Ansprüche 7 oder 8,
**dadurch gekennzeichnet,**
**dass** W und X aromatische Kohlenwasserstoffreste mit 1 bis 50 Kohlenstoffatomen mit einer kovalenten Verknüpfung gemäß Formel IV sind 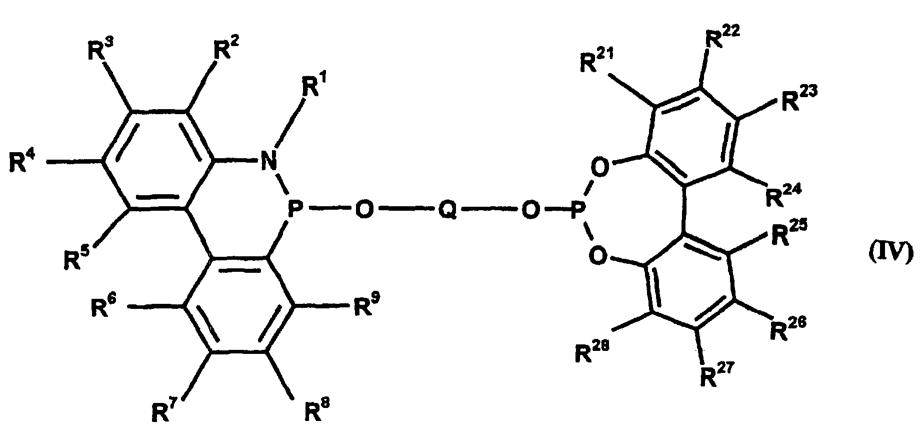 mit
R²¹, R²², R²³, R²⁴, R²⁵, R²⁶, R²⁷, R²⁸ für H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R²¹ bis R²⁸ eine gleiche oder unterschiedliche Bedeutung besitzen und kovalent miteinander verknüpft sein können,
F, Cl, Br, I, -CF₃, -OR²⁹, -COR²⁹, -CO₂R²⁹, -CO₂M, -SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, substituierter oder unsubstituierter, aliphatischer oder aromatischer Kohlenwasserstoffrest mit 1 bis 25 Kohlenstoffatomen,
M = Alkalimetall, Erdalkalimetall, Ammonium, Phosphoniumion,
wobei
R¹, R², R³, R⁴, R⁵, R⁶, R⁷, R⁸, R⁹ und Q die in Anspruch 7 genannten Bedeutungen besitzen.

11. Phosphininmetallkomplex nach einem der Ansprüche 7 bis 8,
**dadurch gekennzeichnet,**
**dass** W und X aromatische Kohlenwasserstoffreste mit 1 bis 50 Kohlenstoffatomen mit kovalenten Verknüpfungen gemäß Formel V 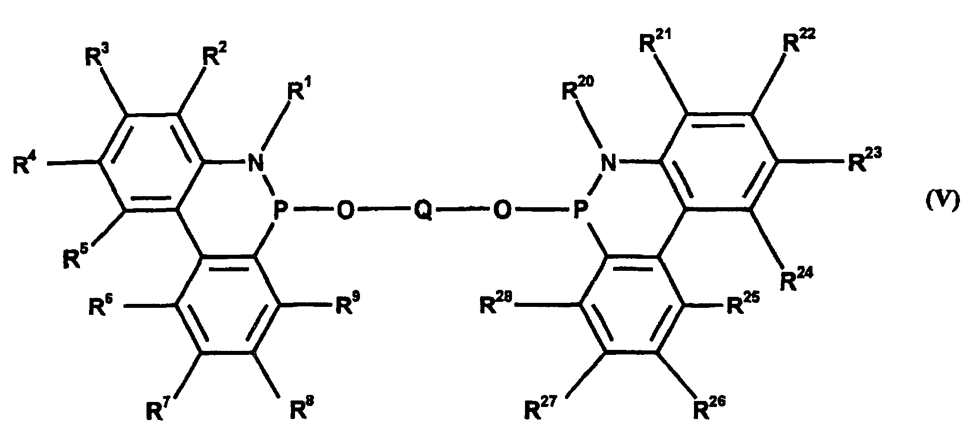 sind,
mit
R²⁰ = H, aliphatischer oder aromatischer Kohlenstoffrest mit 1 bis 25 Kohlenstoffatomen,
R²¹, R²², R²³, R²⁴, R²⁵, R²⁶, R²⁷, R²⁸ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, wobei R²¹ bis R²⁸ eine gleiche oder unterschiedliche Bedeutung besitzen und kovalent miteinander verknüpft sein können,
F, Cl, Br, I, -CF₃, -OR²⁹, -COR²⁹, -CO₂R²⁹, -CO₂M, -SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, substituierter oder unsubstituierter, aliphatischer oder aromatischer Kohlenwasserstoffrest mit 1 bis 25 Kohlenstoffatomen,
M = Alkalimetall, Erdalkalimetall, Ammonium, Phosphoniumion
und
R¹, R², R³, R⁴, R⁵, R⁶, R⁷, R⁸, R⁹ und Q die in Anspruch 7 genannten Bedeutungen besitzen, wobei R² bis R⁹ eine gleiche oder unterschiedliche Bedeutung besitzen und kovalent miteinander verknüpft sein können.

12. Phosphininmetallkomplex nach einem der Ansprüche 7 bis 11,
**dadurch gekennzeichnet,**
**dass** das Q ein Kohlenwasserstoffrest gemäß Formel VI ist 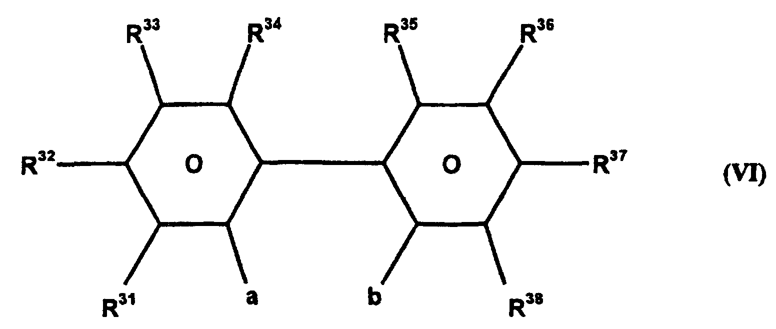 mit
R³¹, R³², R³³, R³⁴, R³⁵, R³⁶, R³⁷, R³⁸ = H, aliphatischer, alicyclischer, aliphatisch-alicyclischer, heterocyclischer, aliphatisch-heterocyclischer, aromatischer, aromatisch-aromatischer, aliphatisch-aromatischer Kohlenwasserstoffrest mit 1 bis 50 Kohlenstoffatomen, F, Cl, Br, I, -CF₃, -OR³⁹, -COR³⁹, -CO₂R³⁹, -CO₂M, - SR³⁹, -SO₂R³⁹, -SOR³⁹, -SO₃R³⁹, -SO₃M, -SO₂NR³⁹R⁴⁰, NR³⁹R⁴⁰, N=CR³⁹R⁴⁰, NH₂,
R³⁹, R⁴⁰ = H, substituierter oder unsubstituierter, aliphatischer oder aromatischer Kohlenwasserstoffrest mit 1 bis 25 Kohlenstoffatomen,
M = Alkalimetall-, Erdalkalimetall-, Ammonium-, Phosphoniumion
wobei die Positionen a und b als Anknüpfpunkte dienen.

13. Phosphininmetallkomplex nach einem der Ansprüche 7 bis 12,
**dadurch gekennzeichnet,**
**dass** als Metall Rhodium, Platin, Kobalt oder Ruthenium eingesetzt wird.

14. Verwendung der Phosphinine gemäß einem der Ansprüche 1 bis 6 zur Hydroformylierung von Olefinen.

15. Verwendung der Phosphininmetallkomplexe gemäß einem der Ansprüche 7 bis 13 zur Hydroformylierung von Olefinen.

16. Verwendung der Phosphinine gemäß einem der Ansprüche 1 bis 6 zur Hydroformylierung von Olefinen unter Anwesenheit von weiteren phosphorhaltigen Liganden.

17. Verwendung der Phosphininmetallkomplexe gemäß einem der Ansprüche 7 bis 13 zur Hydroformylierung von Olefinen unter Anwesenheit von weiteren phosphorhaltigen Liganden.

## Claims

1. A phosphinine of the formula I 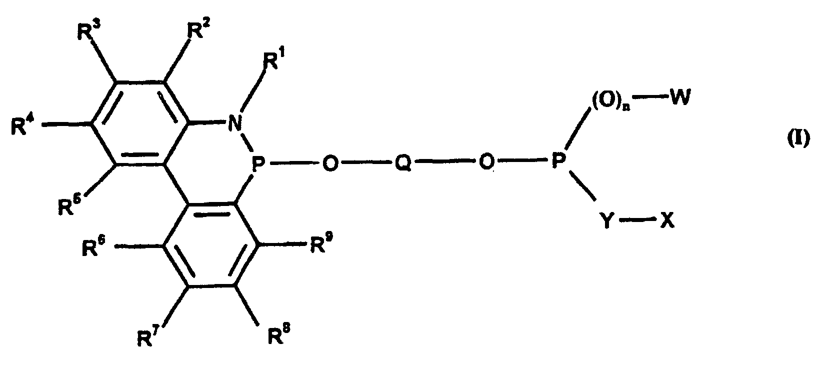 where
n = 0 or 1,
Y = O, NH, NR¹,
R¹ = H, an aliphatic or aromatic hydrocarbon radical having from 1 to 25 carbon atoms,
R², R³, R⁴, R⁵, R⁶, R⁷, R⁸, R⁹ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R² to R⁹ are identical or different and may be covalently linked to one another,
F, Cl, Br, I, -CF₃, -OR¹⁰, -COR¹⁰, -CO₂R¹⁰, -CO₂M, -SR¹⁰, -SO₂R¹⁰, -SOR¹⁰, -SO₃R¹⁰, -SO₃M, -SO₂NR¹⁰R¹¹, NR¹⁰R¹¹, N=CR¹⁰R¹¹, NH₂,
R¹⁰, R¹¹ = H, a substituted or unsubstituted, aliphatic or aromatic hydrocarbon radical having from 1 to 25 carbon atoms, identical or different,
M = an alkali metal, alkaline earth metal, ammonium or phosphonium ion,
Q = a divalent aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms,
W, X = aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, which may be identical or different or covalently linked to one another.

2. A phosphinine according to claim 1, **characterized in that** W and X are aliphatic, alicyclic, aliphatic-alicyclic, heterocyclic, aliphatic-heterocyclic, aromatic, aliphatic-aromatic hydrocarbon radicals having from 1 to 50 carbon atoms and are covalently linked as in formula II 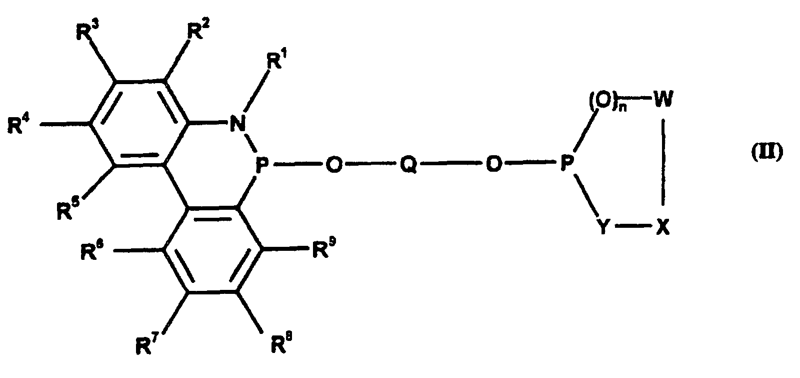 and R¹, R², R³, R⁴, R⁵, R⁶, R⁷, R⁸, R⁹, W, X, Y, n and Q are as defined in claim 1.

3. A phosphinine according to claim 1, **characterized in that** W and X are aromatic hydrocarbon radicals having from 1 to 50 carbon atoms and are covalently linked as in formula III 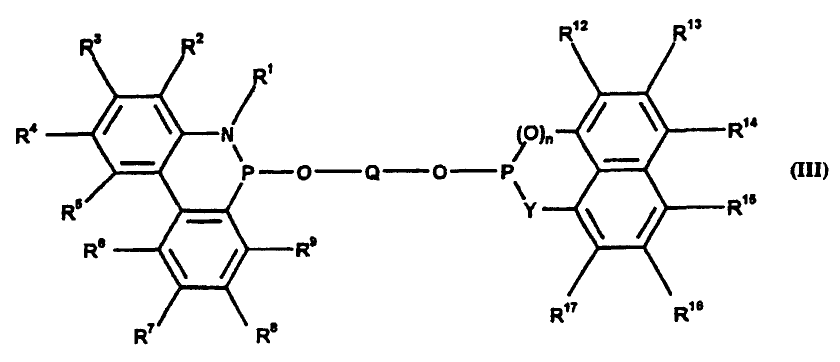 where
R¹², R¹³, R¹⁴, R¹⁵, R¹⁶, R¹⁷ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R¹² to R¹⁷ are identical or different and may be covalently linked to one another,
F, Cl, Br, I, -CF₃, -OR¹⁸, -COR¹⁸, -CO₂R¹⁸, -CO₂M, - SR¹⁸, -SO₂R¹⁸, -SOR¹⁸, -SO₃R¹⁸, -SO₃M, -SO₂NR¹⁸R¹⁹, NR¹⁸R¹⁹, N=CR¹⁸R¹⁹, NH₂,
R¹⁸, R¹⁹ = H, a substituted or unsubstituted, aliphatic or aromatic hydrocarbon radical having from 1 to 25 carbon atoms, identical or different,
M = an alkali metal, alkaline earth metal, ammonium or phosphonium ion and
R¹, R², R³, R⁴, R⁵, R⁶, R⁷, R⁸, R⁹, n, Y and Q are as defined in claim 1.

4. A phosphinine according to either one of claims 1 and 2, **characterized in that** W and X are aromatic hydrocarbon radicals having from 1 to 50 carbon atoms and are covalently linked as in formula IV 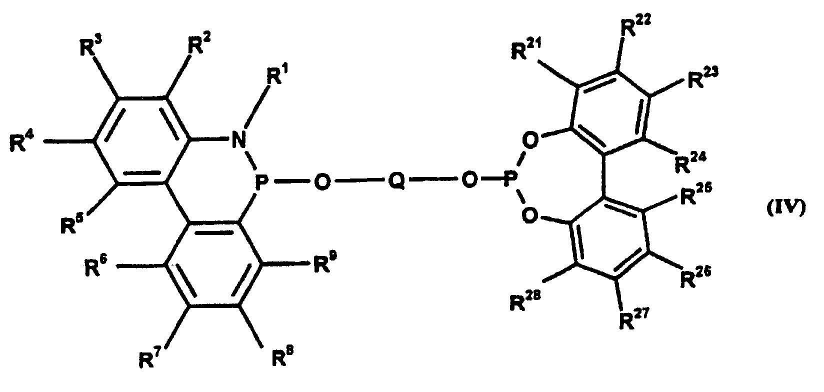 where
R²¹, R²², R²³, R²⁴, R²⁵, R²⁶, R²⁷, R²⁸ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R²¹ to R²⁸ are each identical or different and may be covalently linked to one another,
F, Cl, Br, I, -CF₃, -OR²⁹, -COR²⁹, -CO₂R²⁹, -CO₂M, - SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, a substituted or unsubstituted, aliphatic or aromatic hydrocarbon radical having from 1 to 25 carbon atoms,
M = an alkali metal, alkaline earth metal, ammonium or phosphonium ion,
where
R¹, R², R³, R⁴, R⁵, R⁶, R⁷, R⁸, R⁹ and Q are as defined in claim 1.

5. A phosphinine according to either one of claims 1 and 2, **characterized in that** W and X are aromatic hydrocarbon radicals having from 1 to 50 carbon atoms and are covalently linked as in formula V 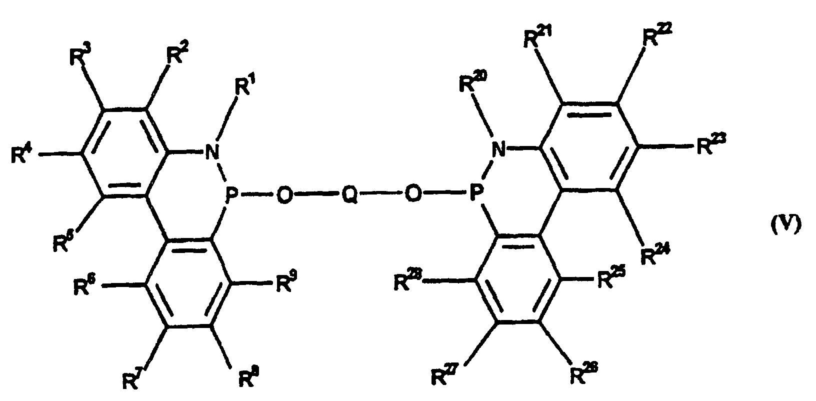 where
R²⁰ = H, an aliphatic or aromatic hydrocarbon radical having from 1 to 25 carbon atoms,
R²¹, R²², R²³, R²⁴, R²⁵, R²⁶, R²⁷, R²⁸ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R²¹ to R²⁸ are identical or different and may be covalently linked to one another,
F, Cl, Br, I, -CF₃, -OR²⁹, -COR²⁹, -CO₂R²⁹, -CO₂M, - SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, a substituted or unsubstituted, aliphatic or aromatic hydrocarbon radical having from 1 to 25 carbon atoms,
M = an alkali metal, alkaline earth metal, ammonium or phosphonium ion,
and
R¹, R², R³, R⁴, R⁵, R⁶, R⁷, R⁸, R⁹ and Q are as defined in claim 1, where R² to R⁹ are identical or different and may be covalently linked to one another.

6. A phosphinine according to any one of claims 1 to 5, **characterized in that** Q is a hydrocarbon radical of the formula VI 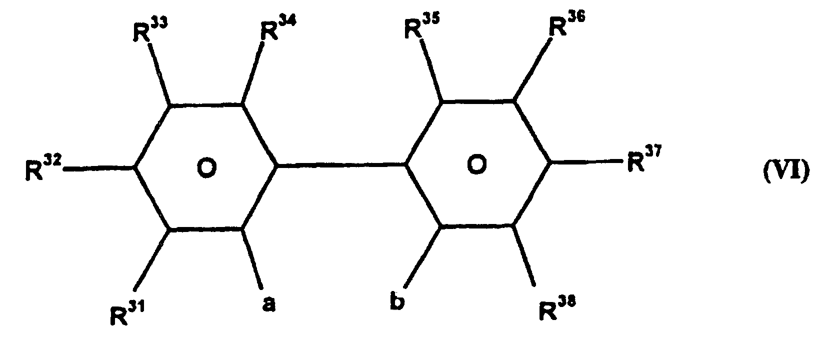 where
R³¹, R³², R³³, R³⁴, R³⁵, R³⁶, R³⁷, R³⁸ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms,
F, Cl, Br, I, -CF₃, -OR³⁹, -COR³⁹, -CO₂R³⁹, -CO₂M, -SR³⁹, -SO₂R³⁹, -SOR³⁹, -SO₃R³⁹, -SO₃M, -SO₂NR³⁹R⁴⁰, NR³⁹R⁴⁰, N=CR³⁹R⁴⁰, NH₂,
R³⁹, R⁴⁰ = H, a substituted or unsubstituted, aliphatic or aromatic hydrocarbon radical having from 1 to 25 carbon atoms,
M = an alkali metal, alkaline earth metal, ammonium or phosphonium ion,
where the positions a and b serve as linkage points.

7. A phosphinine-metal complex comprising a metal of transition group 4, 5, 6, 7 or 8 of the Periodic Table of the Elements and one or more phosphinines of the formula I 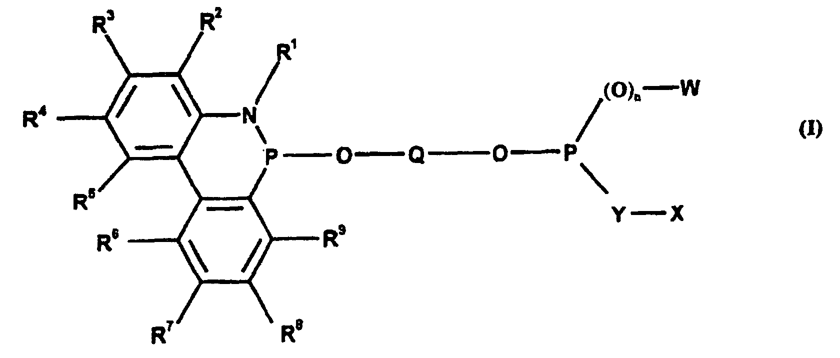 where
n = 0 or 1,
Y = O, NH, NR¹,
R¹ = H, an aliphatic or aromatic hydrocarbon radical having from 1 to 25 carbon atoms,
R², R³, R⁴, R⁵, R⁶, R⁷, R⁸, R⁹ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R² to R⁹ are identical or different and may be covalently linked to one another,
F, Cl, Br, I, -CF₃, -OR¹⁰, -COR¹⁰, -CO₂R¹⁰, -CO₂M, -SR¹⁰, -SO₂R¹⁰, -SOR¹⁰, -SO₃R¹⁰, -SO₃M, -SO₂NR¹⁰R¹¹, NR¹⁰R¹¹, N=CR¹⁰R¹¹, NH₂,
R¹⁰, R¹¹ = H, a substituted or unsubstituted, aliphatic or aromatic hydrocarbon radical having from 1 to 25 carbon atoms, identical or different,
M = an alkali metal, alkaline earth metal, ammonium or phosphonium ion,
Q = a divalent aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms,
W, X = aliphatic, alicyclic, aliphatic-alicyclic, heterocyclic, aliphatic-heterocyclic, aromatic, aromatic-aromatic, aliphatic-aromatic hydrocarbon radicals having from 1 to 50 carbon atoms, which may be identical or different or covalently linked to one another.

8. A phosphinine-metal complex according to claim 7, charcterized in that W and X are aliphatic, alicyclic, aliphatic-alicyclic, heterocyclic, aliphatic-heterocyclic, aromatic, aliphatic-aromatic hydrocarbon radicals having from 1 to 50 carbon atoms and are covalently linked as in formula II 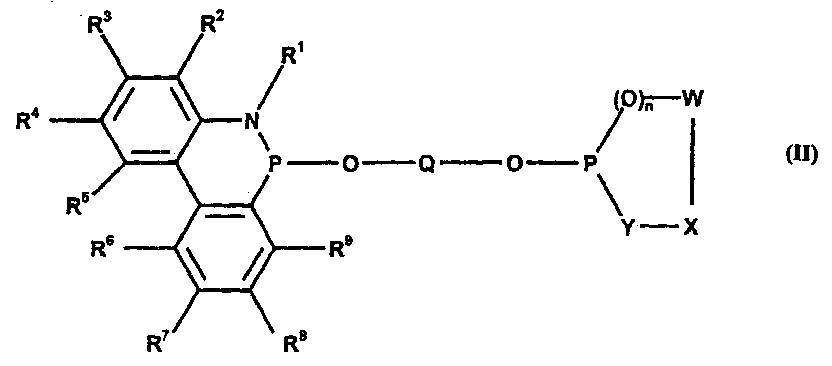 and R¹, R², R³, R⁴, R⁵, R⁶, R⁷, R⁸, R⁹, W, X, Y, n and Q are as defined in claim 1.

9. A phosphinine-metal complex according to claim 7, **characterized in that** W and X are aromatic hydrocarbon radicals having from 1 to 50 carbon atoms and are covalently linked as in formula III 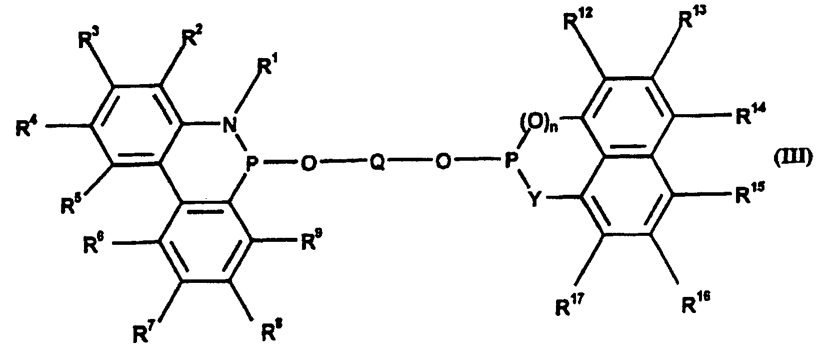 where
R¹², R¹³, R¹⁴, R¹⁵, R¹⁶, R¹⁷ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R¹² to R¹⁷ are identical or different and may be covalently linked to one another,
F, Cl, Br, I, -CF₃, -OR¹⁸, -COR¹⁸, -CO₂R¹⁸, -CO₂M, -SR¹⁸, -SO₂R¹⁸, -SOR¹⁸, -SO₃R¹⁸, -SO₃M, -SO₂NR¹⁸R¹⁹, NR¹⁸R¹⁹, N=CR¹⁸R¹⁹, NH₂,
R¹⁸, R¹⁹ = H, a substituted or unsubstituted, aliphatic or aromatic hydrocarbon radical having from 1 to 25 carbon atoms, identical or different,
M = an alkali metal, alkaline earth metal, ammonium or phosphonium ion and
R¹, R², R³, R⁴, R⁵, R⁶, R⁷, R⁸, R⁹, n, Y and Q are as defined in claim 7.

10. A phosphinine-metal complex according to either one of claims 7 and 8, **characterized in that** W and X are aromatic hydrocarbon radicals having from 1 to 50 carbon atoms and are covalently linked as in formula IV 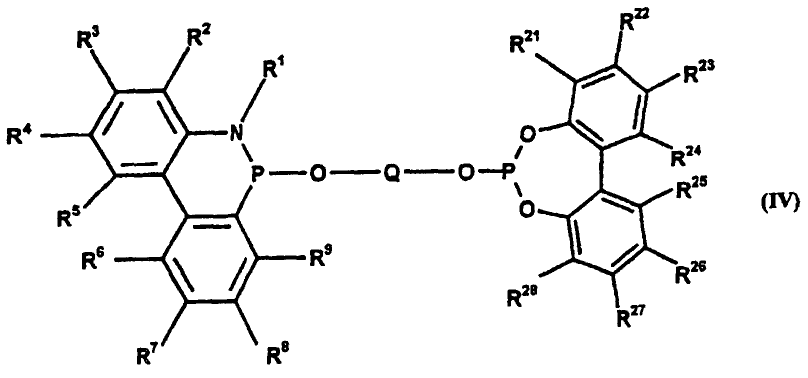 where
R²¹, R²², R²³, R²⁴, R²⁵, R²⁶, R²⁷, R²⁸ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R²¹ to R²⁸ are identical or different and may be covalently linked to one another,
F, Cl, Br, I, -CF₃, -OR²⁹, -COR²⁹, -CO₂R²⁹, -CO₂M, -SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, a substituted or unsubstituted, aliphatic or aromatic hydrocarbon radical having from 1 to 25 carbon atoms,
M = an alkali metal, alkaline earth metal, ammonium or phosphonium ion,
where
R¹, R², R³, R⁴, R⁵, R⁶, R⁷, R⁸, R⁹ and Q are as defined in claim 7.

11. A phosphinine-metal complex according to either one of claims 7 and 8, charcterized in that W and X are aromatic hydrocarbon radicals having from 1 to 50 carbon atoms and are covalently linked as in formula V 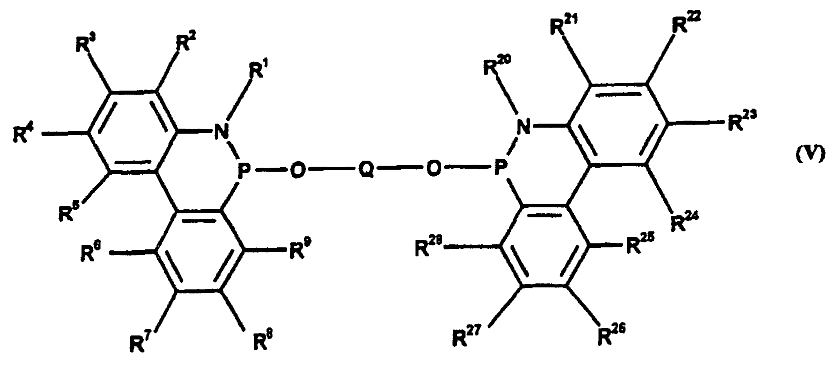 where
R²⁰ = H, an aliphatic or aromatic hydrocarbon radical having from 1 to 25 carbon atoms,
R²¹, R²², R²³, R²⁴, R²⁵, R²⁶, R²⁷, R²⁸ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms, where R²¹ to R²⁸ are identical or different and may be covalently linked to one another,
F, Cl, Br, I, -CF₃, -OR²⁹, -COR²⁹, -CO₂R²⁹, -CO₂M, - SR²⁹, -SO₂R²⁹, -SOR²⁹, -SO₃R²⁹, -SO₃M, -SO₂NR²⁹R³⁰, NR²⁹R³⁰, N=CR²⁹R³⁰, NH₂,
R²⁹, R³⁰ = H, a substituted or unsubstituted, aliphatic or aromatic hydrocarbon radical having from 1 to 25 carbon atoms,
M = an alkali metal, alkaline earth metal, ammonium or phosphonium ion,
and
R¹, R², R³, R⁴, R⁵, R⁶, R⁷, R⁸, R⁹ and Q are as defined in claim 7 , where R² to R⁹ are identical or different and may be covalently linked to one another.

12. A phosphinine-metal complex according to any one of claims 7 to 11, **characterized in that** Q is a hydrocarbon radical of the formula VI 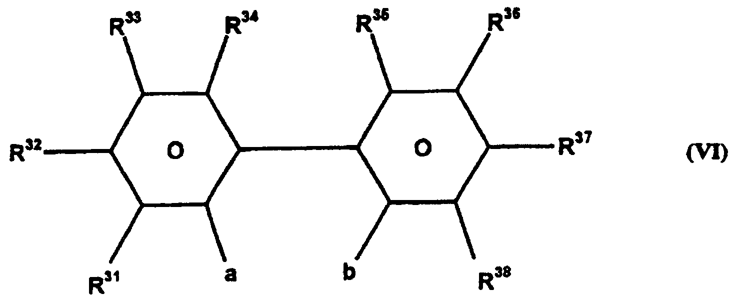 where
R³¹, R³², R³³, R³⁴, R³⁵, R³⁶, R³⁷, R³⁸ = H, an aliphatic, alicyclic, aliphatic-alicyclic, heterocyclic, aliphatic-heterocyclic, aromatic, aromatic-aromatic, aliphatic-aromatic hydrocarbon radical having from 1 to 50 carbon atoms,
F, Cl, Br, I, -CF₃, -OR³⁹, -COR³⁹, -CO₂R³⁹, -CO₂M, -SR³⁹, -SO₂R³⁹, -SOR³⁹, -SO₃R³⁹, -SO₃M, -SO₂NR³⁹R⁴⁰, NR³⁹R⁴⁰, N=CR³⁹R⁴⁰, NH₂,
R³⁹, R⁴⁰ = H, a substituted or unsubstituted, aliphatic or aromatic hydrocarbon radical having from 1 to 25 carbon atoms,
M = an alkali metal, alkaline earth metal, ammonium or phosphonium ion,
where the positions a and b serve as linkage points.

13. A phosphinine-metal complex according to any one of claims 7 to 12, **characterized in that** the metal used is rhodium, platinum, cobalt or ruthenium.

14. The use of a phosphinine according to any one of claims 1 to 6 for the hydroformylation of olefins.

15. The use of a phosphinine-metal complex according to any one of claims 7 to 13 for the hydroformylation of olefins.

16. The use of a phosphinine according to any one of claims 1 to 6 for the hydroformylation of olefins in the presence of further phosphorus-containing ligands.

17. The use of a phosphinine-metal complex according to any one of claims 7 to 13 for the hydroformylation of olefins in the presence of further phosphorus-containing ligands.

## Revendications

1. Phosphinine de Formule 1 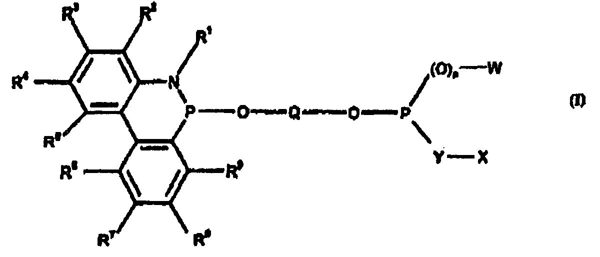 avec
n = 0 ou 1
Y = 0, NH, NR¹
R¹ = H, un reste hydrocarboné aliphatique ou aromatique ayant de 1 à 25 atomes de carbone,
R², R³, R⁴, R⁵, R⁶, R⁷, R⁸, R⁹ = H, reste hydrocarboné aliphatique alicyclique, aliphatico-alicyclique, hétérocyclique, aliphatico-hétérocyclique, aromatique, aromatico-aromatique, aliphatico-aromatique, ayant de 1 à 50 atomes de carbone, dans lesquels R² à R⁹ peuvent posséder une signification identique ou différente et être couplés les uns avec les autres d'une manière covalente, F, Cl, Br, I, -CF₃, -OR¹⁰, -COR¹⁰, -CO₂R¹⁰, -CO₂M, -SR¹⁰, -SO₂R¹⁰, -SOR¹⁰, -SO₃R¹⁰, -SO₃M, -SO₂NR¹⁰R¹¹, NR¹⁰ R¹¹, N = CR¹⁰ R¹¹, NH₂,
R¹⁰, R¹¹ = H, un reste hydrocarboné substitué ou non substitué, aliphatique ou aromatique ayant de 1 à 25 atomes de carbone ayant une signification identique ou différente,
M = un ion de métal alcalin, métal alcalino-terreux, ammonium ou phosphonium,
Q = un reste hydrocarboné aliphatique, alicyclique, aliphatico-alicyclique, hétérocyclique aliphatico-hétérocyclique, aromatique, aromatico-aromatique, aliphatico-aromatique, bivalents ayant de 1 à 50 atomes de carbone,
W, X = un reste hydrocarboné aliphatique alicyclique, aliphatico-alicyclique, hétéro-cyclique, aliphatico-hétérocyclique, aromatique, aromatico-aromatique, aliphatico-aromatique ayant de 1 à 50 atomes de carbone, qui peuvent être identiques ou différents, ou être couplés l'un à l'autre d'une manière covalente.

2. Phosphinine conformément à la revendication 1,
**caractérisée en ce que**
W et X sont des restes hydrocarbonés aliphatiques, alicycliques, aliphatico-alicycliques, hétérocyclique, aliphatico-hétérocycliques aromatiques, aliphatico-aromatiques, ayant de 1 à 50 atomes de carbone, ayant un couplage covalent conformément à la formule II, 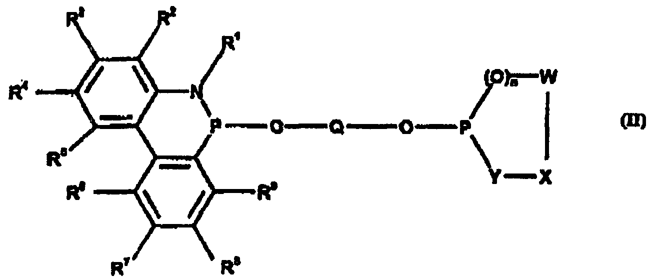 et R¹, R², R³, R⁴, R⁵, R⁶, R⁷, R⁸, R⁹, W, X, Y, n et Q possèdent les significations mentionnées à la revendication 1.

3. Phosphinine conformément à la revendication 1,
**caractérisée en ce que**
W et X sont des restes hydrocarbonés aromatiques ayant de 1 à 50 atomes de carbone avec des couplages covalents conformément à la formule III. 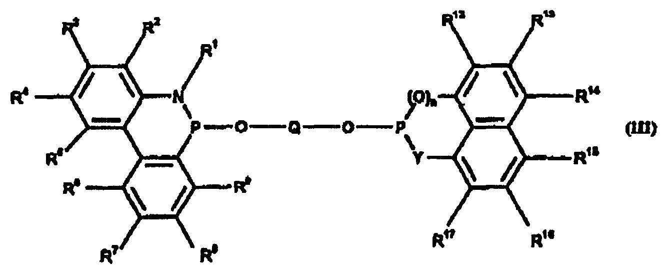 avec R¹², R¹³, R¹⁴, R1⁵, R¹⁶, R¹⁷ = H, un reste hydrocarboné aliphatique, alicyclique, aliphatico-alicyclique, hétérocyclique, aliphatique hétérocylique, aromatique, aromatico-aromatique, aliphatico-aromatique, ayant de 1 à 50 atomes de carbone, pour lesquels R¹² à R¹⁷ possèdent une signification identique ou différente et peuvent être couplés les uns aux autres d'une manière covalente, F, Cl, Br, i, -CF₃, -OR¹⁸, -COR¹⁸, -CO₂R¹⁸, -CO₂^{M}, -SR¹⁸, -SO₂R¹⁸, SOR¹⁸, SO₃R¹⁸, SO₃M, SO₂NR¹⁸R¹⁹, NR¹⁸R¹⁹, N = CR¹⁸R¹⁹, NH₂,
R¹⁸, R¹⁹ = H ou un reste hydrocarboné substitué ou non substitué, aliphatique ou aromatique, ayant de 1 à 25 atomes de carbone, avec une signification identique ou différente,
M est un ion de métal alcalin, de métal alcalino-terreux, d'ammonium, de phosphonium et
R¹, R², R³, R⁴, R⁵, R⁶, R⁷, R⁸, R⁹, n, y et Q possèdent les significations mentionnées à la revendication 1.

4. Phosphinine selon l'une des revendications 1 ou 2,
**caractérisée en ce que**
W et X sont des restes hydrocarbonés aromatiques ayant de 1 à 50 atomes de carbone avec un couplage covalent conformément à la formule IV, 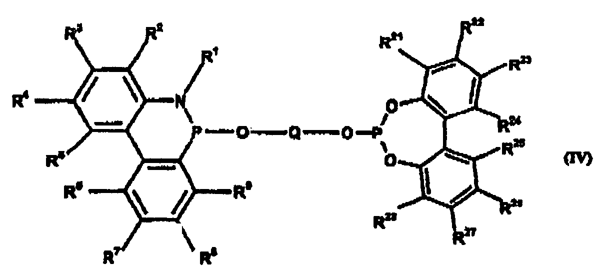 avec R²¹, R²², R²³, R²⁴, R²⁵, R²⁶, R²⁷ et R²⁸ représentent de l'hydrogène, un reste hydrocarboné aliphatique, alicyclique, aliphatico alicyclique, hétérocyclique, aliphatico-hétérocyclique, aromatique, aromatico-aromatique, aliphatico-aromatique, ayant de 1 à 50 atomes de carbone, pour lesquels R²¹ à R²⁸ possèdent une signification identique ou différente et peuvent être couplés les uns aux autres d'une manière covalente,
F, Cl, Br, i, -CF₃, -OR²⁹, -COR²⁹, -CO₂R²⁹, -CO₂M,
- SR²⁹, -SO₂R²⁹, -SOR²⁹, -SO₃R²⁹, -SO₃M, -SO₂NR²⁹R³⁰, NR²⁹R³⁰
N = CR²⁹R³⁰, NH₂,
R^{29,} R³⁰ = H, un reste hydrocarboné substitué ou non substitué, aliphatique ou aromatique ayant de 1 à 25 atomes de carbone,
M = métal alcalin, métal alcalino-terreux, ammonium, un ion phosphonium,
dans lesquels R¹, R², R³, R⁴, R⁵, R⁶, R⁷, R⁸, R⁹ et Q possèdent les significations mentionnées à la revendication 1.

5. Phosphinine selon l'une des revendications 1 à 2,
**caractérisée en ce que**
W et X sont des restes hydrocarbonés aromatiques ayant de 1 à 50 atomes de carbone avec des couplages covalents, conformément à la formule V. 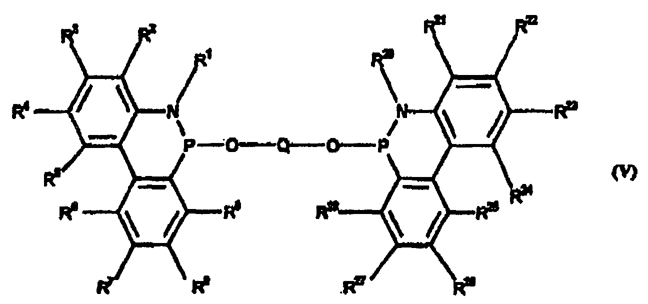 avec R²⁰ = H, un reste hydrocarboné aliphatique ou aromatique ayant de 1 à 25 atomes de carbone,
R²¹, R²², R²³, R²⁴, R²⁵, R²⁶, R²⁷, R²⁸ = H, un reste hydrocarboné aliphatique, alicyclique aliphatico-alicyclique, hétérocyclique, aliphatico-hétérocyclique, aromatique, aromatico-aromatique, aliphatico-aromatique, ayant de 1 à 50 atomes de carbone, pour lesquels R²¹ à R²⁸ possèdent une signification identique ou différente et peuvent être coupplés l'un à l'autre de manière covalente.
F, CI, Br, I, - CF₃, - OR²⁹, - COR²⁹, - CO₂R²⁹, - CO₂M, - SR²⁹, - SO₂R²⁹, - SOR²⁹, -SO₃R²⁹, SO₃M, -SO₂NR²⁹R³⁰, NR²⁹R³⁰, N = CR²⁹R³⁰, NH₂,
R²⁹, R³⁰ = H, un reste hydrocarboné substitué ou non substitué aliphatique ou aromatique ayant de 1 à 25 atomes de carbone,
M = métal alcalin, métal alcaline-terreux ammonium, un ion phosphonium et
R¹, R², R³, R⁴, R⁵, R⁶, R⁷, R⁸, R⁹ et Q possèdent les significations mentionnées à la revendication 1, pour lesquelles R² à R⁹ possèdent une signification identique ou différente et peuvent être couplés l'un à l'autre d'une manière covalente.

6. Phosphinine selon l'une des revendications 1 à 5,
**caractérisée en ce que**
Q est un reste hydrocarboné conformément à la formule VI. 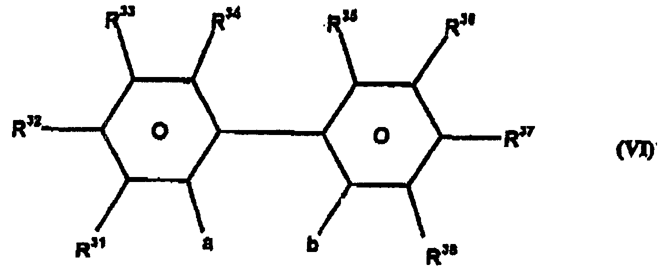 avec R³¹, R³², R³³, R³⁴, R³⁵, R³⁶, R³⁷, R³⁸ = H, un reste hydrocarboné aliphatique, alicyclique, aliphatico-alicyclique, hétérocyclique, aliphatico-hétérocyclique, aromatique, aromatico-aromatique, aliphatico-aromatique, ayant de 1 à 50 atomes de carbone,
F, Cl, Br, I, - CF₃, -OR³⁹, -COR³⁹, -CO₂R³⁹, -CO₂M, -SR³⁹, -SO₂R³⁹, - SOR³⁹, -SO₃R³⁹, -SO₃M, -SO₂NR³⁹R⁴⁰, NR³⁹R⁴⁰, N = CR³⁹R⁴⁰, NH₂,
R³⁹, R⁴⁰ = H, reste hydrocarboné substitué ou non substitué, aliphatique ou aromatique, ayant de 1 à 25 atomes de carbone.
M = ion de métal alcalin, de métal alcalino-terreux d'ammonium, dephosphonium dans lesquels les positions a et b servent de points de couplage.

7. Complexe métal phosphinine contenant un métal du 4ème, 5ème, 6ème, 7ème ou 8ème sous-groupe du système périodique des éléments et une ou plusieurs phosphinines de formule I, 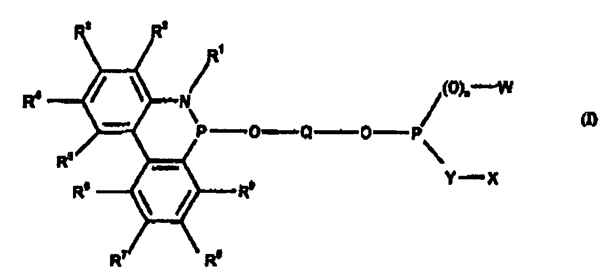 avec n = 0 ou 1
Y = O, NH, NR¹
R¹ = H, reste hydrocarboné aliphatique ou aromatique ayant de 1 à 25 atomes de carbone, R², R³, R⁴, R⁵, R⁶, R⁷, R⁸, R⁹ = H, reste hydrocarboné aliphatique ou aromatique ayant 1 à 25 atomes de carbone,
R², R³, R⁴, R⁵, R⁶, R⁷, R⁸, R⁹ = H, reste hydrocarboné aliphatique, alicyclique, aliphatico-alicyclique, hétérocyclique, aliphatico-hétérocyclique, aromatique, aromatico-aromatique, aliphatico-aromatique, ayant de 1 à 50 atomes de carbone, pour lesquels R² à R⁹ possèdent une signification identique ou différente, et peuvent être couplés l'un à l'autre d'une manière covalente,
F, Cl, Br, I, - CF³, - OR¹⁰, - COR¹⁰, - CO₂R¹⁰, -CO₂M - SR¹⁰, - SO₂R¹⁰, -SOR¹⁰, -SO₃R¹⁰, - SO₃M, - SO₂NR¹⁰R¹¹, NR¹⁰R¹¹, N = CR¹⁰R¹¹, NH₂.
R¹⁰, R¹¹ = H, un reste hydrocarboné substitué ou non substitué, aliphatique ou aromatique, ayant de 1 à 25 atomes de carbone avec une signification identique ou différente,
M = ion de métal alcalin, de métal alcalino-terreux, d'ammonium, de phosphonium,
Q = reste hydrocarboné aliphatique, alicyclique, aliphatico-alicyclique, hétérocyclique, aliphatico-hétérocyclique, aromatique, aromatico-aromatique, aliphatico-aromatique, divalent ayant de 1 à 50 atomes de carbone,
W, X = des restes aliphatiques, aliphatiques aliphatico-alicycliques, hétérocycliques, aliphatico-hétérocycliques, aromatiques, aromatico-aromatiques, aliphatico-aromatiques, ayant de 1 à 50 atomes de carbone, qui peuvent être identiques ou différents ou être couplés l'un à l'autre d'une manière covalente.

8. Complexe métalphosphinine selon la revendication 7,
**caractérisé en ce que**
W et X sont des restes hydrocarbonés aliphatiques, alicycliques, aliphatico-alicycliques, hétérocycliques, aliphatico-hétérocycliques, aromatiques, aliphatico-aromatiques, ayant de 1 à 50 atomes de carbone, avec un couplage covalent conformément à la formule II, 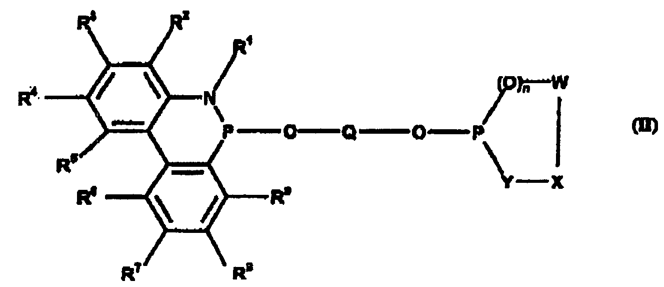 Et R¹, R², R³, R⁴, R⁵, R⁶, R⁷, R⁸, R⁹, W, X, Y, n et Q possèdent les significations mentionnées à la revendication 1.

9. Complexe métal/phosphinine selon la revendication 7,
**caractérisé en ce que**
W et X sont des restes hydrocarbonés aromatiques ayant de 1 à 50 atomes de carbone avec des couplages covalents conformément à la formule III, 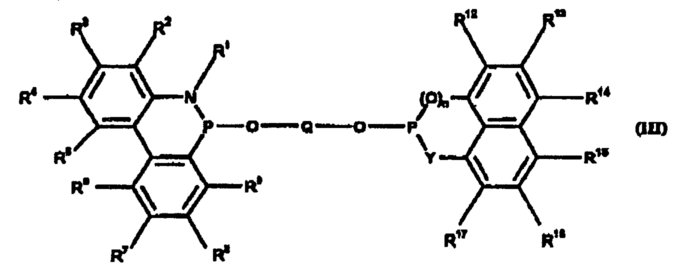 avec R¹², R¹³, R¹⁴, R¹⁵, R¹⁶, R¹⁷ = hydrogène ou reste hydrocarboné aliphatique, alicyclique, aliphatico-alicyclique, hétérocyclique aliphatico-hétérocyclique, aromatique, aromatico-aromatique, aliphatico-aromatique, ayant de 1 à 50 atomes de carbone, pour lesquels R12 à R17 possèdent une signification identique ou différente et peuvent être couplés l'un à l'autre d'une manière covalente.
F, Cl, Br, I, - CF3, - OR¹⁸, - COR¹⁸, - CO₂R¹⁸, - CO₂^{M}, - SR¹⁸, - SO₂R¹⁸, -SOR¹⁸, - SO₃R¹⁸, - SO₃M, - SO₂NR¹⁸R¹⁹, NR¹⁸R¹⁹, N = CR¹⁸R¹⁹, NH₂,
R¹⁸, R¹⁹ = H, un reste hydrocarboné substitué ou non substitué, aliphatique ou aromatique ayant de 1 à 25 atomes de carbone ayant une signification identique ou différente,
M = ion de métal alcalin, de métal alcalino-terreux, d'ammonium, de phosphonium et R¹, R², R³, R⁴, R⁵, R⁶, R⁷, R⁸, R⁹, n, Y et Q possèdent les significations mentionnées à la revendication 7.

10. Complexe métal-phosphonine selon l'une des revendications 7 ou 8,
**caractérisé en ce que**
W et X sont des restes hydrocarbonés aromatiques ayant dé: 1 à 50 atomes de carbone avec un couplage covalent, conformément à la formule IV, 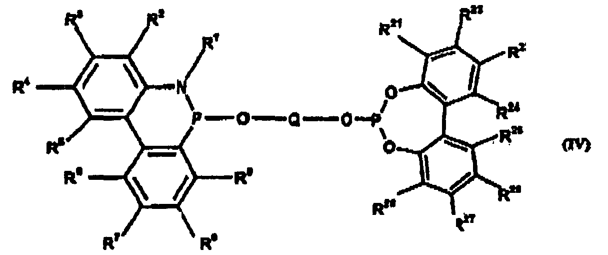 avec R²¹, R²², R²³, R²⁴, R²⁵, R²⁶, R²⁷, R²⁸ représentent de l'hydrogène, un reste hydrocarboné aliphatique, alicyclique, aliphatico-alicyclique, hétérocyclique, aliphatico-hétérocyclique, aromatique, aromatico-aromatique, aliphatico-aromatique, ayant de 1 à 50 atomes de carbone pour laquelle R²¹ à R²⁸ possèdent une signification identique ou différente et peuvent être couplés l'un à l'autre d'une manière covalente,
F, Cl, Br, I, - CF₃, - OR²⁹, - COR²⁹, - CO₂R²⁹, - CO₂M, - SR²⁹, - SO₂R²⁹, - SOR²⁹, - SO₃R²⁹, - SO₃M, - SO₂NR²⁹R³⁰, NR²⁹R³⁰, N = CR²⁹R³⁰, NH₂,
R²⁹, R³⁰ = H, reste hydrocarboné substitué ou non substitué, aliphatique ou aromatique, ayant de 1 à 25 atomes de carbone,
M = métal alcalin, métal alcalino-ferreux, ammonium, ion phosphonium,
pour lesquels R¹, R², R³, R⁴, R⁵, R⁶, R⁷, R⁸, R⁹ et Q possèdent les significations mentionnées à la revendication 7.

11. Complexe métal-phosphinine selon l'une des revendications 7 à 8,
**caractérisé en ce que**
W et X sont des restes hydrocarbonés aromatiques ayant de 1 à 50 atomes de carbone avec des couplages covalents conformément à la formule V, 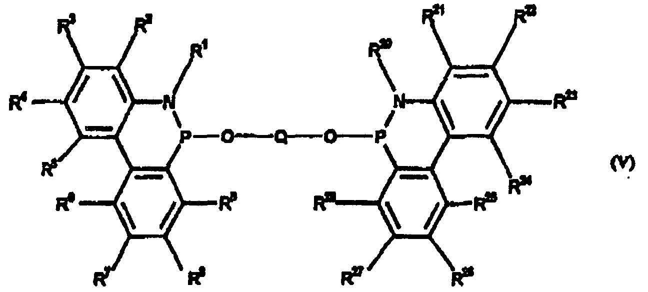 avec R²⁰ = H, reste carboné aliphatique ou aromatique ayant de 1 à 25 atomes de carbone,
R²¹, R²², R²³, R²⁴, R²⁵, R²⁶, R²⁷, R²⁸ = H, reste hydrocarboné aliphatique, alicyclique, aliphatico-alicyclique, hétérocyclique, aliphatico-hétérocyclique, aromatique, aromatico-aromatique, aliphatico-aromatique, ayant de 1 à 50 atomes de carbone, pour lesquels R²¹ à R²⁸ possèdent une signification identique ou différente et peuvent être couplés l'un à l'autre d'une manière covalente,
F, Cl, Br, I, - CF₃ ; - OR²⁹, - COR²⁹, - CO₂R²⁹, - CO₂M, - SR²⁹, -SO₂R²⁹, - SOR²⁹, - SO₃R²⁹, - SO₃M, - SO₂NR²⁹R³⁰, NR²⁹R³⁰, N = CR²⁹R³⁰, NH₂,
R²⁹, R³⁰ = H, un reste hydrocarboné substitué ou non substitué, aliphatique ou aromatique, ayant de 1 à 25 atomes de carbone,
M = métal alcalin, métal alcaline-terreux, ammonium, ion phosphonium, et
R¹, R², R³, R⁴, R⁵, R⁶, R⁷, R⁸, R⁹ et Q possèdent les significations mentionnées à la revendication 7, pour lesquels R² à R⁹ possèdent une signification identique ou différente et peuvent être couplés l'un avec l'autre d'une manière covalente.

12. Complexe métal-phosphinine, selon l'une des revendications 7 à 11,
**caractérisé en ce que**
Q est un reste hydrocarboné conformément à la formule VI, 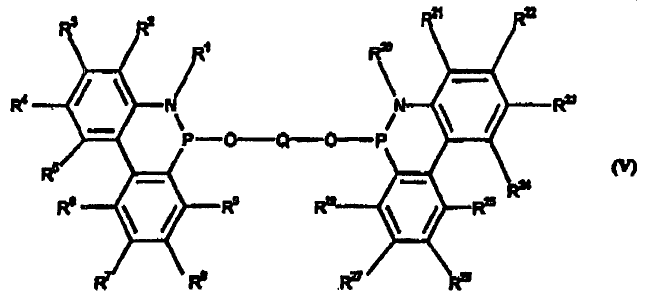 avec R³¹, R³², R³³, R³⁴, R³⁵, R³⁶, R³⁷, R³⁸ = H, un reste hydrocarboné aliphatique, alicyclique, aliphatico-alicyclique, hétérocyclique, aliphatico-hétérocyclique, aromatique, aromatico-aromatique, aliphatico-aromatique, ayant de 1 à 50 atomes de carbone,
F, Cl, Br, I, -CF₃ ; -OR³⁹, -COR³⁹, -CO₂R³⁹, -CO₂M, -SR³⁹, -SO₂R³⁹, SOR³⁹, -SO₃R³⁹, -SO₃M, -SO₂NR³⁹R⁴⁰, NR³⁹R⁴⁰, N = CR³⁹R⁴⁰, NH₂,
R³⁹, R⁴⁰ = H, un reste hydrocarboné substitué ou non substitué, aliphatique ou aromatique ayant de 1 à 25 atomes de carbone,
M = ion de métal alcalin, de métal alcalino-terreux, d'ammonium, de phosphonium,
pour lesquels les positions a et b servent comme points de rattachement.

13. Complexe métal-phosphinine selon l'une des revendications 7 à 12
**caractérisé en ce que** ce que
on met en oeuvre comme métal le rhodium, le platine, le cobalt, ou le ruthénium.

14. Utilisation des phosphinines conformément à l'une des revendications 1 à 6, en vue de l'hydroformylation des oléfines.

15. Utilisation des complexes de métal-phosphinine conformément à l'une des revendications 7 à 13, en vue de l'hydroformylation d'oléfines.

16. Utilisation des phosphinines conformément à l'une des revendications 1 à 6, pour l'hydroformylation d'oléfines en présence d'autres ligands contenant du phosphore.

17. Utilisation des complexes métal-phosphinine conformément â l'une des revendications 7 à 13, pour l'hydroformylation des oléfines en présence d'autres ligands contenant du phosphore.
